# EUROPEAN PATENT APPLICATION

(11) **EP 2 336 178 A1**
(43) Date of publication of application: **22.06.2011**
(21) Application number: 10010299.5
(22) Date of filing: 10.12.2004
(51) Int. Cl.: C07K 16/18, A61K 38/03

(54) **Methods and compositions for inhibiting C-Met dimerization and activation**

(30) Priority: 11.12.2003 US 528909 P
(62) Divisional of application: 04813660.0
(71) Applicant: Genentech, Inc., South San Francisco CA 94080-4990 (US)
(72) Inventor: Wickramasinghe, Dineli M., San Francisco, CA 94114 (US); Kong-Beltran, Monica, San Mateo, CA 94402 (US)
(74) Representative: Woolley, Lindsey Claire

(57) **Abstract**

The invention provides methods and compositions for modulating the HGF/c-met signaling pathway, in particular by regulating c-met dimerization and/or binding of ligand to c-met using a c-met antagonist that disrupts c-met multimerization.

## Description

### RELATED APPLICATIONS

This application is a non-provisional application filed under 37 CFR 1.53(b)(1), claiming priority benefit of provisional application number 60/528,909 filed December 11, 2003, the content of which is incorporated herein in its entirety by reference.

### TECHNICAL FIELD

The present invention relates generally to the fields of molecular biology and growth factor regulation. More specifically, the invention concerns modulators of the HGF/c-met signaling pathway, and uses of said modulators.

### BACKGROUND

HGF is a mesenchyme-derived pleiotrophic factor with mitogenic, motogenic and morphogenic activities on a number of different cell types. HGF effects are mediated through a specific tyrosine kinase, c-met, and aberrant HGF and c-met expression are frequently observed in a variety of tumors. See, e.g., Maulik et al., Cytokine & Growth Factor Reviews (2002), 13:41-59; Danilkovitch-Miagkova & Zbar, J. Clin. Invest. (2002), 109(7):863-867. Regulation of the HGF/c-Met signaling pathway is implicated in tumor progression and metastasis. See, e.g., Trusolino & Comoglio, Nature Rev. (2002), 2:289-300).

HGF binds the extracellular domain of the Met receptor tyrosine kinase (RTK) and regulates diverse biological processes such as cell scattering, proliferation, and survival. HGF-Met signaling is essential for normal embryonic development especially in migration of muscle progenitor cells and development of the liver and nervous system (Bladt et al., 1995; Harnanoue et al., 1996; Maina et al., 1996; Schmidt et al., 1995; Uehara et al., 1995). Developmental phenotypes of Met and HGF knockout mice are very similar suggesting that HGF is the cognate ligand for the Met receptor (Schmidt et al., 1995; Uehara et al., 1995). HGF-Met also plays a role in liver regeneration, angiogenesis, and wound healing (Bussolino et al., 1992; Matsumoto and Nakamura, 1993; Nusrat et al., 1994). The precursor Met receptor undergoes proteolytic cleavage into an extracellular α subunit and membrane spanning β subunit linked by disulfide bonds (Tempest et al., 1988). The β subunit contains the cytoplasmic kinase domain and harbors a multi-substrate docking site at the C-terminus where acapter proteins bind and initiate signaling (Bardelli et al., 1997; Nguyen et al., 1997; Pelicci et al.,1995; Ponzetto et al.,1994; Weidner et al., 1996). Upon HGF binding, activation of Met leads to tyrosine phosphorylation and downstream signaling through Gab1 and Grb2/Sos mediated PI3-kinase and Ras/MAPK activation respectively, which drives cell motility and proliferation (Furge et al., 2000; Hartmann et al., 1994; Ponzetto et al., 1996; Royal and Park, 1995).

Met was shown to be transforming in a carcinogen-treated osteosarcoma cell line (Cooper et al., 1984; Park et al., 1986). Met overexpression or gene-amplification has been observed in a variety of human cancers. For example, Met protein is overexpressed at least 5-fold in colorectal cancers and reported to be gene-amplified in liver metastasis (Di Renzo et al., 1995; Liu et al., 1992). Met protein is also reported to be overexpressed in oral squamous cell carcinoma, hepatocellular carcinoma, renal cell carcinoma, breast carcinoma, and lung carcinoma (Jin et al., 1997; Morello et al., 2001; Natali et al., 1996; Olivero et al., 1996; Suzuki et al., 1994). In addition, overexpression of mRNA has been observed in hepatocellular carcinoma, gastric carcinoma, and colorectal carcinoma (Boix et al., 1994; Kuniyasu et al., 1993; Liu et al., 1992).

A number of mutations in the kinase domain of Met have been found in renal papillary carcinoma which leads to constitutive receptor activation (Olivero et al., 1999; Schmidt et al., 1997; Schmidt et al., 1999). These activating mutations confer constitutive Met tyrosine phosphorylation and result in MAPK activation, focus formation, and tumorigenesis (Jeffers et al., 1997). In addition, these mutations enhance cell motility and invasion (Giordano et al., 2000; Lorenzato et al., 2002). HGF-dependent Met activation in transformed cells mediates increased motility, scattering, and migration which eventually leads to invasive tumor growth and metastasis (Jeffers et al., 1996; Meiners et al., 1998).

Met has been shown to interact with other proteins that drive receptor activation, transformation, and invasion. In neoplastic cells, Met is reported to interact with α6β4 integrin, a receptor for extracellular matrix (ECM) components such as laminins, to promote HGF-dependent invasive growth (Trusolino et al., 2001). In addition, the extracellular domain of Met has been shown to interact with a member of the semaphorin family, plexin B1, and to enhance invasive growth (Giordano et al., 2002). Furthermore, CD44v6, which has been implicated in tumorigenesis and metastasis, is also reported to form a complex with Met and HGF and result in Met receptor activation (Orian-Rousseau et al., 2002).

Met is a member of the subfamily of receptor tyrosine kinases (RTKs) which include Ron and Sea (Maulik et al., 2002). Prediction of the extracellular domain structure of Met suggests shared homology with the semaphorins and plexins. The N-terminus of Met contains a Sema domain of approximately 500 amino acids that is conserved in all semaphorins and plexins. The semaphorins and plexins belong to a large family of secreted and membrane-bound proteins first described for their role in neural development (Van Vactor and Lorenz, 1999). However, more recently semaphorin overexpression has been correlated with tumor invasion and metastasis. A cysteine-rich PSI domain (also referred to as a Met Related Sequence domain) found in plexins, semaphorins, and integrins lies adjacent to the Sema domain followed by four IPT repeats that are immunoglobulin-like regions found in plexins and transcription factors. A recent study suggests that the Met Sema domain is sufficient for HGF and heparin binding (Gherardi et al., 2003).

The role of the Met kinase domain has been investigated in detail, but the extracellular domain of Met is poorly characterized. Although it is known that HGF binds the extracellular domain of Met resulting in receptor activation, it is not clear which sub-domain(s), if any, contribute to receptor dimerization. Nonetheless, it is evident that elucidation of the contribution, if any, of sub-domains within the Met extracellular domain to activation of the HGF/c-met signaling axis would provide significant advantages for the design of targeted therapeutics, for example and in particular in clinical scenarios involving aberrations in the function of the Met receptor itself.

All references cited herein, including patent applications and publications, are incorporated by reference in their entirety.

### DISCLOSURE OF THE INVENTION

The invention is in part based on the demonstration that dimerization of a receptor of hepatocyte growth factor, c-met, is a biological/cellular process that presents as an important and advantageous therapeutic target. It is herein demonstrated that disruption of this process effectively inhibits activation of a cell signaling pathway associated with cell growth dysregulation in numerous disease states, such as cancer. The invention is further based on the finding that certain but not all domains within the c-met protein are necessary for, and/or play a dominant role in, effecting c-met dimerization and hence activation of the HGF/c-met signaling axis. The invention also provides compositions and methods based on interfering with HGF binding to a specific domain in the extracellular portion of c-met. The identification of specific portion(s) of c-met as being necessary for either c-met dimerization or cognate ligand binding to c-met provides a unique and advantageous target for greater fine-tuning in designing prophylatic and/or therapeutic approaches against pathological conditions associated with abnormal or unwanted signaling of the HGF/c-met pathway. Accordingly, the invention provides methods, compositions, kits and articles of manufacture related to modulating the HGF/c-met pathway, including modulation of c-met ligand binding, c-met dimerization, activation, and other biological/physiological activities associated with HGF/c-met signaling.

In one aspect, the invention provides c-met antagonists that disrupt the HGF/c-met signaling pathway. For example, the invention provides a c-met antagonist that disrupts c-met dimerization. In one embodiment, a c-met antagonist of the invention disrupts dimerization function of c-met Sema domain (i.e., ability of Sema domain to function in effecting receptor dimerization). In one example, a c-met antagonist interferes with ability of c-met Sema domain to effect c-met dimerization. Interference can be direct or indirect. For example, a c-met antagonist may bind to a sequence within the c-met Sema domain, and thereby inhibit interaction of said bound domain with its binding partner (such as another c-met molecule). In another example, a c-met antagonist may bind to a sequence that is not within the c-met Sema domain, but wherein said binding results in disruption of the ability of the c-met Sema domain to interact with its binding partner (such as another c-met molecule). In one embodiment, an antagonist of the invention binds to c-met (e.g., the extracellular domain) such that c-met dimerization is disrupted. In one embodiment, an antagonist of the invention binds to c-met such that ability of c-met Sema domain to effect c-met dimerization is disrupted. For example, in one embodiment, the invention provides an antagonist which upon binding to a c-met molecule inhibits dimerization of said molecule. In one embodiment, a c-met antagonist of the invention specifically binds a sequence in the c-met Sema domain.

In one embodiment, an antagonist of the invention disrupts c-met dimerization comprising homodimerization. In one embodiment, an antagonist of the invention disrupts c-met dimerization comprising heterodimerization (i.e., c-met dimerization with a non-c-met molecule).

In some instances, it may be advantageous to have a c-met antagonist that does not interfere with binding of a ligand (such as HGF) to c-met. Accordingly, in some-embodiments, an antagonist of the invention does not bind a ligand (such as HGF) binding site on c-met. In another embodiment, an antagonist of the invention does not substantially inhibit ligand (e.g., HGF) binding to c-met. In one embodiment, an antagonist of the invention does not substantially compete with a ligand (e.g., HGF) for binding to c-met. In one example, an antagonist of the invention can be used in conjunction with one or more other antagonists, wherein the antagonists are targeted at different processes and/or functions within the HGF/c-met axis. Thus, in one embodiment, a c-met antagonist of the invention binds to an epitope on c-met distinct from an epitope to which another c-met antagonist, such as the Fab fragment of the monoclonal antibody produced by the hybridoma cell line deposited under American Type Culture Collection Accession Number ATCC HB-11894 (hybridoma 1A3.3.13) or HB-11895 (hybridoma 5D5.11.6), binds. In another embodiment, a c-met antagonist of the invention is distinct from (i.e., it is not) a Fab fragment of the monoclonal antibody produced by the hybridoma cell line deposited under American Type Culture Collection Accession Number ATCC HB-11894 (hybridoma 1A3.3.13) or HB-11895 (hybridoma 5D5.11.6). In one embodiment, a c-met antagonist of the invention does not comprise a c-met binding sequence of an antibody produced by the hybridoma cell line deposited under American Type Culture Collection Accession Number ATCC Hub-11894 (hybridoma 1A3.3.13) or HB-11895 (hybridoma 5D5.11.6).

In some instances, it may be advantageous to have a c-met antagonist that disrupts both c-met dimerization and ligand binding. For example, an antagonist of the invention may further comprise an ability to compete with HGF for binding to c-met.

In one embodiment of a c-met antagonist of the invention, binding of the antagonist to c-met inhibits c-met activation by HGF. In one embodiment of a c-met antagonist of the invention, binding of the antagonist to c-met in a cell inhibits proliferation, scattering, morphogenesis and/or motility of the cell.

In one embodiment, a c-met antagonist of the invention comprises a peptide comprising at least a portion of c-met Sema domain or variant thereof. In one example, an antagonist of the invention comprises a peptide comprising at least one of the sequences selected from the group consisting of LDAQT (SEQ ID NO:1) (e.g., residues 269-273 of c-met), LTEKRKKRS (SEQ ID NO:2) (e.g., residues 300-308 of c-met), KPDSAEPM (SEQ ID NO:3) (e.g., residues 350-357 of c-met) and NVRCLQHF (SEQ ID NO:4) (e.g., residues 381-388 of c-met). In another example, an antagonist of the invention comprises a peptide consisting essentially of at least one of the sequences selected from the group consisting of LDAQT (SEQ ID NO:1), LTEKRKKRS (SEQ ID NO:2), KPDSAEPM (SEQ ID NO:3) and NVRCLQHF (SEQ ID NO:4). In one embodiment, said peptide comprises the sequence LTEKRKKRS (SEQ ID NO:2). In one embodiment, said peptide consists essentially of LTEKRKKRS (SEQ ID NO:2). In one embodiment, said peptide comprises the sequence LDAQT (SEQ ID NO: 1). In one embodiment, said peptide consists essentially of LDAQT (SEQ ID NO: 1). In one embodiment, said peptide comprises the sequence KPDSAEPM (SEQ ID NO:3). In one embodiment, said peptide consists essentially of KPDSAEPM (SEQ ID NO:3). In one embodiment, said peptide comprises the sequence NVRCLQHF (SEQ ID NO:4). In one embodiment, said peptide consists essentially of NVRCLQHF (SEQ ID NO:4). In some embodiments, the peptide is a variant comprising an amino acid sequence having at least 50%, 60%, 70%, 80%, 90%, 95%, 98% sequence identity or similarity with the sequence LDAQT (SEQ ID NO: 1), LTEKRKKRS (SEQ ID NO:2), KPDSAEPM (SEQ ID NO:3) and/or NVRCLQHF (SEQ ID NO:4), wherein the variant retains ability to form a complex with a c-met molecule. In some embodiments, these peptides comprise modifications that enhance their inhibitory and/or therapeutic effect (including, e.g., enhanced affinity, improved pharmacokinetics properties (such as half life, stability, clearance rate), reduced toxicity to the subject). Such modifications include, e.g., modifications involving glycosylation, pegylation, substitution with non-naturally occurring but functionally equivalent amino acid, linking groups, etc. Suitable modifications are well known in the art, and furthermore can be determined empirically as necessary.

In some embodiments, a c-met antagonist of the invention is or comprises a small molecule (for e.g, an organic molecule), peptide (e.g., an oligopeptide), antibody, antibody fragment, aptamer, oligonucleotide (e.g., antisense oligonucleotide), or a combination thereof.

In some embodiments, a c-met antagonist of the invention is obtained by a screening or identification method of the invention as described herein.

In another aspect, the invention provides methods for screening for or identifying a c-met antagonist. In one example, said methods comprise contacting a candidate substance with a cell that expresses c-met, whereby inhibition of c-met dimerization and/or activation in said cell (as assessed by any of a variety of criteria and methods, some of which are described herein) in the presence of said candidate substance indicates that the substance is a c-met antagonist. In another example, said methods comprise contacting a candidate substance with a target molecule comprising at least a portion of c-met Sema domain, whereby a substance that specifically binds said target molecule is selected (as a c-met antagonist). In some embodiments, screening methods of the invention further comprise contacting a selected substance with a cell expressing c-met, wherein inhibition of c-met dimerization in the cell is assessed (e.g., wherein extent of c-met dimerization in said cell is detected or quantitated). Inhibition of c-met dimerization can be assayed in a variety of ways known in the art, and based on any of a variety of criteria known in the art, some of which are described in greater detail herein. For example, inhibition of c-met dimerization may be indicated by a decrease in amount of c-met activation, which may in turn be indicated by, for instance, amount of c-met associated cell signaling within a cell. Cell signaling can be assessed by a variety of methods and based on a variety of criteria, which are known in the art, some of which are described herein. For example, occurrence of cell signaling in the HGF/c-met pathway can manifest biologically in the form of change in phosphorylation of target molecules in the signaling pathway. Thus, e.g., amount of protein phosphorylation associated with one or more known phosphorylation targets in the HGF/c-met pathway could be measured. Examples of such phosphorylation targets include c-met itself and mitogen activated protein kinase (MAPK).

In one aspect, the invention provides compositions comprising one or more antagonists of the invention and a carrier. In one embodiment, the carrier is pharmaceutically acceptable.

In one aspect, the invention provides nucleic acids encoding a c-met antagonist of the invention. In one embodiment, a nucleic acid of the invention encodes a c-met antagonist which is or comprises a polypeptide (e.g., an oligopeptide). In one embodiment, a nucleic acid of the invention encodes a c-met antagonist which is or comprises an antibody or fragment thereof.

In one aspect, the invention provides vectors comprising a nucleic acid of the invention.

In one aspect, the invention provides host cells comprising a nucleic acid or a vector of the invention. A vector can be of any type, for example a recombinant vector such as an expression vector. Any of a variety of host cells can be used. In one embodiment, a host cell is a prokaryotic cell, for example, E. *coli.* In one embodiment, a host cell is a eukaryotic cell, for example a mammalian cell such as Chinese Hamster Ovary (CHO) cell.

In one aspect, the invention provides methods for making an antagonist of the invention. For example, the invention provides a method of making a c-met antagonist which is or comprises an antibody (or fragment thereof), said method comprising expressing in a suitable host cell a recombinant vector of the invention encoding said antibody (or fragment thereof), and recovering said antibody. In another example, the invention provides a method of making a c-met antagonist which is or comprises a polypeptide (such as an oligopeptide), said method comprising expressing in a suitable host cell a recombinant vector of the invention encoding said polypeptide (such as an oligopeptide), and recovering said polypeptide (such as an oligopeptide).

In one aspect, the invention provides an article of manufacture comprising a container, and a composition contained within the container, wherein the composition comprises one or more c-met antagonists of the invention. In one embodiment, the composition comprises a nucleic acid of the invention. In one embodiment, a composition comprising antagonist further comprises a carrier, which in some embodiments is pharmaceutically acceptable. In one embodiment, an article of manufacture of the invention further comprises instructions for administering the composition (e.g., the antagonist) to a subject.

In one aspect, the invention provides a kit comprising a first container comprising a composition comprising one or more c-met antagonists of the invention; and a second container comprising a buffer. In one embodiment, the buffer is pharmaceutically acceptable. In one embodiment, a composition comprising antagonist further comprises a carrier, which in some embodiments is pharmaceutically acceptable. In one embodiment, a kit further comprises instructions for administering the composition (e.g., the antagonist) to a subject.

In one aspect, the invention provides use of a c-met antagonist of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder. The c-met antagonist can be of any form described herein, including antibody, antibody fragment, small molecule (e.g., an organic molecule), polypeptide (e.g., an oligopeptide), nucleic acid (e.g., an oligonucleotide, such as an antisense oligonucleotide), an aptamer, or combination thereof.

In one aspect, the invention provides use of a nucleic acid of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of an expression vector of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of a host cell of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of an article of manufacture of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of a kit of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

The invention provides methods and compositions useful for modulating disease states associated with dysregulation of the HGF/c-met signaling axis. The HGF/c-met signaling pathway is involved in multiple biological and physiological functions, including, e.g., cell proliferation and angiogenesis. Thus, in one aspect, the invention provides a method comprising administering to a subject a substance/molecule that modulates function of a sub-domain of the extracellular portion of c-met, whereby HGF/c-met signaling is modulated. In one embodiment, the sub-domain comprises all or at least a portion of c-met Sema domain.

In one aspect, the invention provides a method of inhibiting c-met activated cell proliferation, said method comprising contacting a cell or tissue with an effective amount of a c-met antagonist of the invention, whereby cell proliferation associated with c-met activation is inhibited.

In one aspect, the invention provides a method of treating a pathological condition associated with dysregulation of c-met activation in a subject, said method comprising administering to the subject an effective amount of a c-met antagonist of the invention, whereby said condition is treated.

In one aspect, the invention provides a method of inhibiting the growth of a cell that expresses c-met or hepatocyte growth factor, or both, said method comprising contacting said cell with a c-met antagonist of the invention thereby causing an inhibition of growth of said cell. In one embodiment, the cell is contacted by HGF expressed by a different cell (e.g., through a paracrine effect).

In one aspect, the invention provides a method of therapeutically treating a mammal having a cancerous tumor comprising a cell that expresses c-met or hepatocyte growth factor, or both, said method comprising administering to said mammal an effective amount of a c-met antagonist of the invention, thereby effectively treating said mammal. In one embodiment, the cell is contacted by HGF expressed by a different cell (e.g., through a paracrine effect).

In one aspect, the invention provides a method for treating or preventing a cell proliferative disorder associated with increased expression or activity of c-met or hepatocyte growth, or both, said method comprising administering to a subject an effective amount of a c-met antagonist of the invention, thereby effectively treating or preventing said cell proliferative disorder. In one embodiment, said proliferative disorder is cancer.

In one aspect, the invention provides a method for inhibiting the growth of a cell, wherein growth of said cell is at least in part dependent upon a growth potentiating effect of c-met or hepatocyte growth factor, or both, said method comprising contacting said cell with an effective amount of a c-met antagonist of the invention, thereby inhibiting the growth of said cell. In one embodiment, the cell is contacted by HGF expressed by a different cell (e.g., through a paracrine effect).

A method of therapeutically treating a tumor in a mammal, wherein the growth of said tumor is at least in part dependent upon a growth potentiating effect of c-met or hepatocyte growth factor, or both, said method comprising contacting said cell with an effective amount of a c-met antagonist of the invention, thereby effectively treating said tumor. In one embodiment, the cell is contacted by HGF expressed by a different cell (e.g., through a paracrine effect).

Methods of the invention can be used to affect any suitable pathological state, for example, cells and/or tissues associated with dysregulation of the HGF/c-met signaling pathway. In one embodiment, a cell that is targeted in a method of the invention is a cancer cell. For example, a cancer cell can be one selected from the group consisting of a breast cancer cell, a colorectal cancer cell, a lung cancer cell, a papillary carcinoma cell (e.g., of the thyroid gland), a colon cancer cell, a pancreatic cancer cell, an ovarian cancer cell, a cervical cancer cell, a central nervous system cancer cell, an osteogenic sarcoma cell, a renal carcinoma cell, a hepatocellular carcinoma cell, a bladder cancer cell, aprostate cancer cell, a gastric carcinoma cell, a head and neck squamous carcinoma cell, a lymphoma cell, a melanoma cell and a leukemia cell. In one embodiment, a cell that is targeted in a method of the invention is a hyperproliferative and/or hyperplastic cell. In one embodiment, a cell that is targeted in a method of the invention is a dysplastic cell. In yet another embodiment, a cell that is targeted in a method of the invention is a metastatic cell.

Methods of the invention can further comprise additional treatment steps. For example, in one embodiment, a method further comprises a step wherein a targeted cell and/or tissue (e.g., a cancer cell) is exposed to radiation treatment and/or a chemotherapeutic agent.

As described herein, c-met activation is an important biological process the dysregulation of which leads to numerous pathological conditions. Accordingly, in one embodiment of methods of the invention, a cell that is targeted (e.g., a cancer cell) is one in which activation of c-met is enhanced as compared to a normal cell of the same tissue origin. In one embodiment, a method of the invention causes the death of a targeted cell. For example, contact with an antagonist of the invention may result in a cell's inability to signal through the c-met pathway, which results in cell death or inhibition of cell growth.

Dysregulation of c-met activation (and thus signaling) can result from a number of cellular changes, including, for example, overexpression of HGF (c-met's cognate ligand) and/or c-met itself. Accordingly, in some embodiments, a method of the invention comprises targeting a cell wherein c-met or hepatoctye growth factor, or both, is more abundantly expressed by said cell (e.g., a cancer cell) as compared to a normal cell of the same tissue origin. A c-met-expressing cell can be regulated by HGF from a variety of sources, i.e. in an autocrine or paracrine manner. For example, in one embodiment of methods of the invention, a targeted cell is contacted/bound by hepatocyte growth factor expressed in/by a different cell (e.g., via a paracrine effect). Said different cell can be of the same or of a different tissue origin relative to a targeted cell. In one embodiment, a targeted cell is contacted/bound by HGF expressed by the targeted cell itself (e.g., via an autocrine effect/loop). C-met activation and/or signaling can also occur independent of ligand. Hence, in one embodiment of methods of the invention, c-met activation in a targeted cell occurs independent of ligand.

### BRIEF DESCRIPTION OF THE DRAWINGS

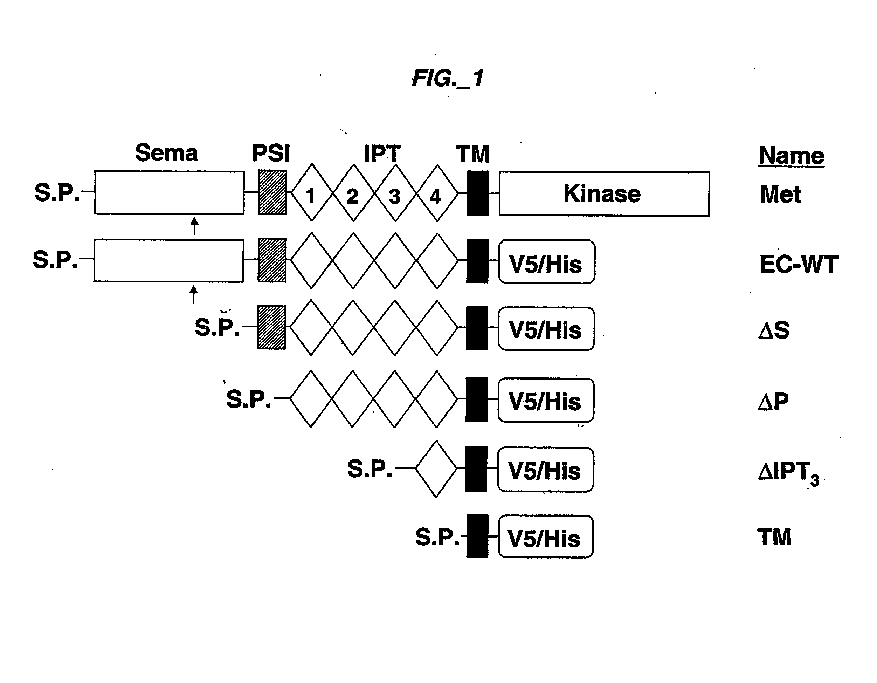

### Figure 1. Schematic representation of Met deletion mutants

Sub-domain deletions of the Met extracellular domain were made from the N-terminus and tagged after the TM region with V5/His. The Met signal peptide (S.P.) sequence was appended to the N-terminus of each mutant. The abbreviations are the following: Sema-semaphorin; PSI-plexin, semaphorin, integrin; IPT-immunoglobulin-like regions in plexins and transcription factors; and TM-transmembrane. The arrow on the Sema region points to the Met proteolytic site.

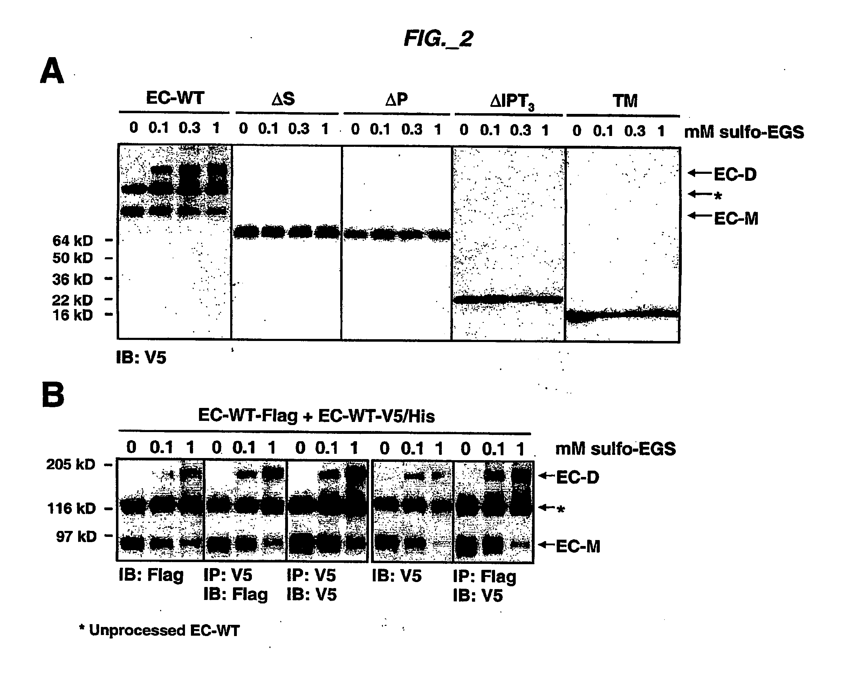

### Figure 2. The Sema domain is necessary for Met crosslinking

**A:** The Met deletion mutants were transfected into 293 cells and exposed to increasing concentrations of sulfo-EGS. 10 µg of lysates were analyzed by 4-12% SDS-PAGE and immunoblotted with V5 antibody. EC-M represents monomeric EC-WT and EC-D refers to dimeric EC-WT. The asterisk indicates the presence of unprocessed EC-WT.
**B:** EC-WT-Flag and EC-WT-V5/His were cotransfected in 293 cells, subjected to increasing concentrations of sulfo-EGS, lysed, immunoprecipitated with either V5 or Flag antibodies and analyzed by 8% SDS-PAGE. The immunoprecipitated samples were blotted with either Flag or V5 antibodies.

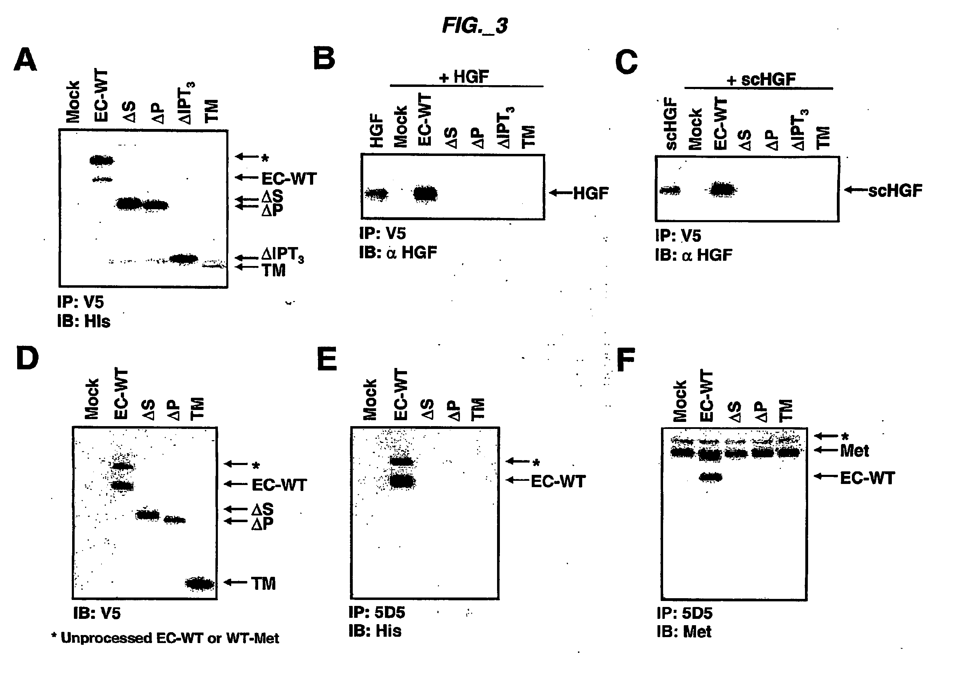

### Figure 3. HGF and an anti-Met antibody (referred to herein as "5D5") bind the Sema domain of Met

**A:** 293 cells were transfected with the indicated Met V5/His tagged construct, immunoprecipitated with V5 antibody, analyzed by 4-12% SDS-PAGE, and immunoblotted with His antibody.
**B:** The immunoprecipitates from **A** were incubated with 5 µg HGF. The samples were analyzed by 4-12% SDS-PAGE and immunoblotted with HGF antibody that recognizes the α chain. 10 ng of input HGF served as a positive control.
**C:** Immunoprecipitations and immunoblotting were performed as in **B** using scHGF (R494E). As a positive control, 15 ng scHGF (R494E) was analyzed with the samples.
**D:** 293 cells were transfected with the indicated constructs were analyzed by 4-12% SDS-PAGE and immunoblotted with V5 antibody.
**E:** The transfected lysates from **D** were immunoprecipitated with anti-Met 5D5 antibody and immunoblotted with His antibody.
**F:** The nitrocellulose membrane from **B** was reprobed with Met C-12 antibody.

### Figure 4. Endogenous c-Met crosslinks in tumor cell lines

Increasing concentrations of sulfo-EGS were added to 293, H441, MDA-MB-435, and A549 cells that had been serum-starved. A549 cells were also incubated with 100 µg/ml HGF prior to crosslinking. Lysates were immunoblotted with Met C-12 antibody. Bands indicated with an * represent unprocessed WT-Met.

### Figure 5. Met signal transduction is inhibited by rSema and anti-Met 5D5-Fab. The extracellular domain of Met attenuates Met signal transduction

**A:** A549 cells were kept serum-free and treated with 0, 5, 10, 50, or 100 µg/ml of rSema with 10 ng/ml HGF for 10 min. for phospho-Met and phospho-MAPK analysis. Lysates were immunoprecipitated with Met C-12 antibody and immunoblotted with phosphotyrosine antibodies to detect phosphorylated Met followed by immunoblotting with Met antibody. H441 and MDA-MB-435 cells were treated with 0, 5, 10, or 100 µg/ml rSema plus 5 or 10 ng/ml HGF, respectively, for 5 min. Proteins were detected with phospho-MAPK and reprobed with MAPK antibodies. Bands indicated with an ^{*} represent unprocessed WT-Met.
**B:** The data in **A** were quantified using NIH Image software and are represented as bar graphs in the left panels comparing the ratio of phosphorylated to unphosphorylated proteins between each sample. The right panels represent data from another experiment.
**C:** A549, H441, and MDA-MB-435 cells were kept serum-free and treated with 10 µg/ml rSema or anti-Met 5D5-Fab. The samples were immunoblotted with phosphotyrosine, Met, phospho-MAPK, or MAPK antibodies. Bands indicated with an * represent unprocessed WT-Met.
**D:** The data in **C** were quantified and presented as in **B**.
**E:** A549 cells were transfected with the indicated constructs. The expression of the Met mutants was detected by immunoblotting with V5 antibody. Bands indicated with an ^{*} represent unprocessed EC-WT.
F: (Left) Lysates from E were immunoprecipitated with Met C-12 antibody and immunoblotted with phospho-tyrosine antibody. The membrane was reprobed with Met antibody. (Right) Lysates were immunoblotted with phospho-MAPK and then reprobed with MAPK.
**G:** The data in **F** were quantified using NIH Image software and is represented as bar graphs in the left panels comparing the ratio of phosphorylated to unphosphorylated proteins for each sample. The right panels represent data from another experiment. Bars in each graph, from left to right, correspond to lanes designated in **F** as "Mock", "EC-WT"', "ΔS", "ΔP" and "TM", respectively.

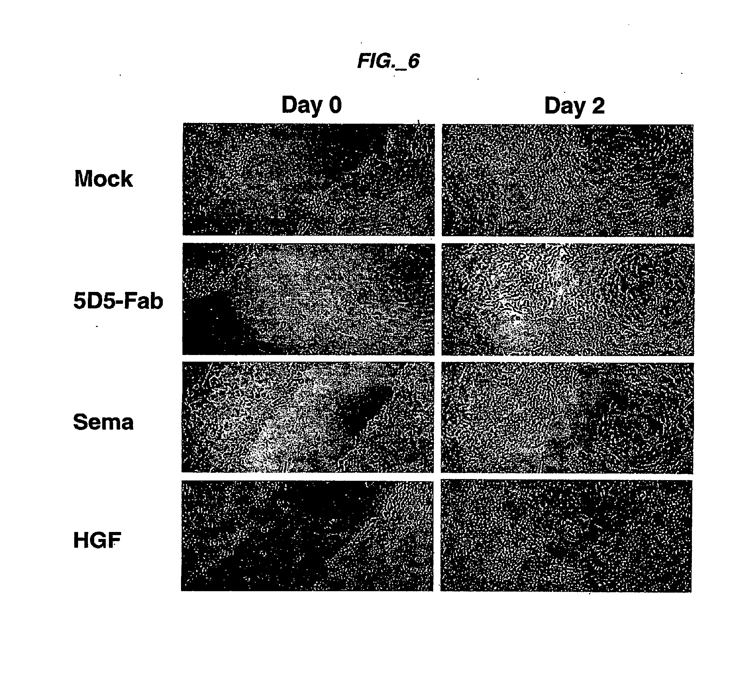

### Figure 6. rSema and anti-Met 5D5-Fab inhibit cell motility

H441 cells were scraped and treated with mock, rSema, anti-Met 5D5-Fab, or HGF in serum-free media over the course of two days. Four independent experiments were carried out and a representative data set is shown.

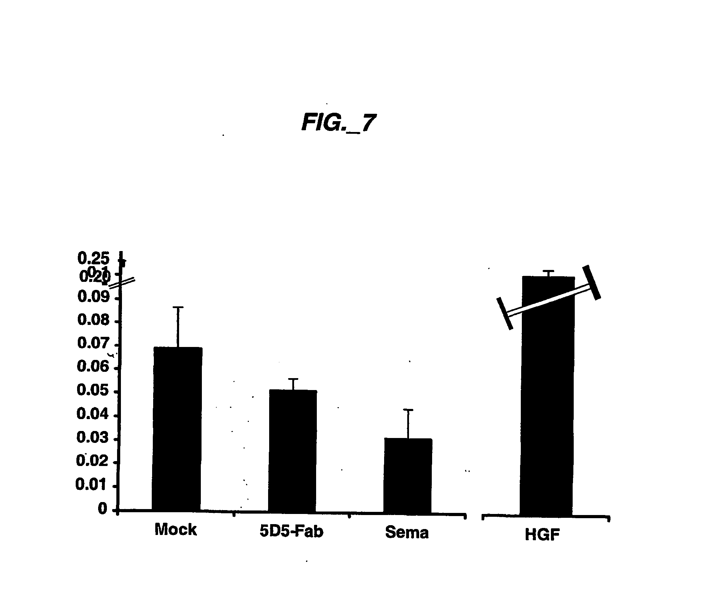

### Figure 7. Cell migration is inhibited by rSema and anti-Met 5D5-Fab

MDA-MB-435 cells were seeded in the upper chamber of each transwell in serum-free media and treated with mock, rSema, or anti-Met 5D5-Fab. As a positive control, HGF was added to the lower chamber in mock treated wells. The migrating cells were stained with crystal-violet and the absorbance of eluted cells was measured at 560 nm. Three independent migration studies were carried out in duplicate and a representative graph is shown.

### MODES FOR CARRYING OUT THE INVENTION

The invention provides methods of interfering with the HGF/c-met signaling axis to treat and/or prevent pathological conditions associated with dysregulation of said axis. The invention is based at least in part on the elucidation of the subdomains/subsequences within c-met demonstrated herein to be necessary for ligand binding and/or c-met receptor dimerization (and thus, c-met activation). The invention further provides compositions, kits and articles of manufacture useful in methods of the invention.

Details of these methods, compositions, kits and articles of manufacture are provided herein.

### General Techniques

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed., 1994); "A Practical Guide to Molecular Cloning" (Perbal Bernard V., 1988).

### Definitions

"Percent (%) amino acid sequence identity" with respect to a peptide (e.g., VDWVCFRDLGCDWEL, LDAQT, LTEKRKKRS, KPDSAEPM, NVRCLQHF) or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table A below. The ALIGN-2 sequence comparison computer program was authored by Genentech, Inc. and the source code shown in Table A below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table A below. The ALIGN-2 program should be compiled for use on a UNIX operating system, preferably digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

Unless specifically stated otherwise, all % amino acid sequence identity values used herein are obtained as described in the immediately preceding paragraph using the ALIGN-2 computer program.

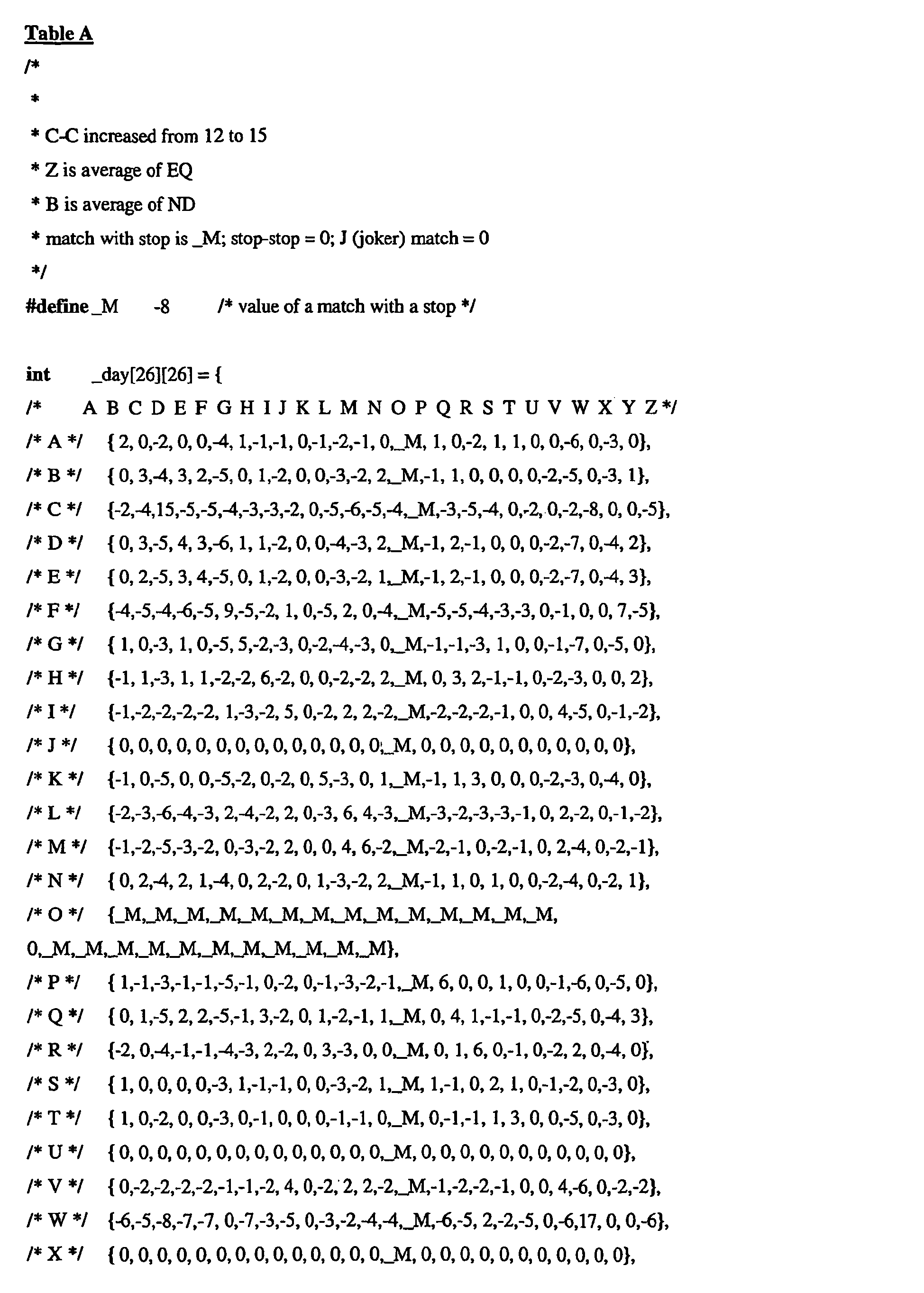

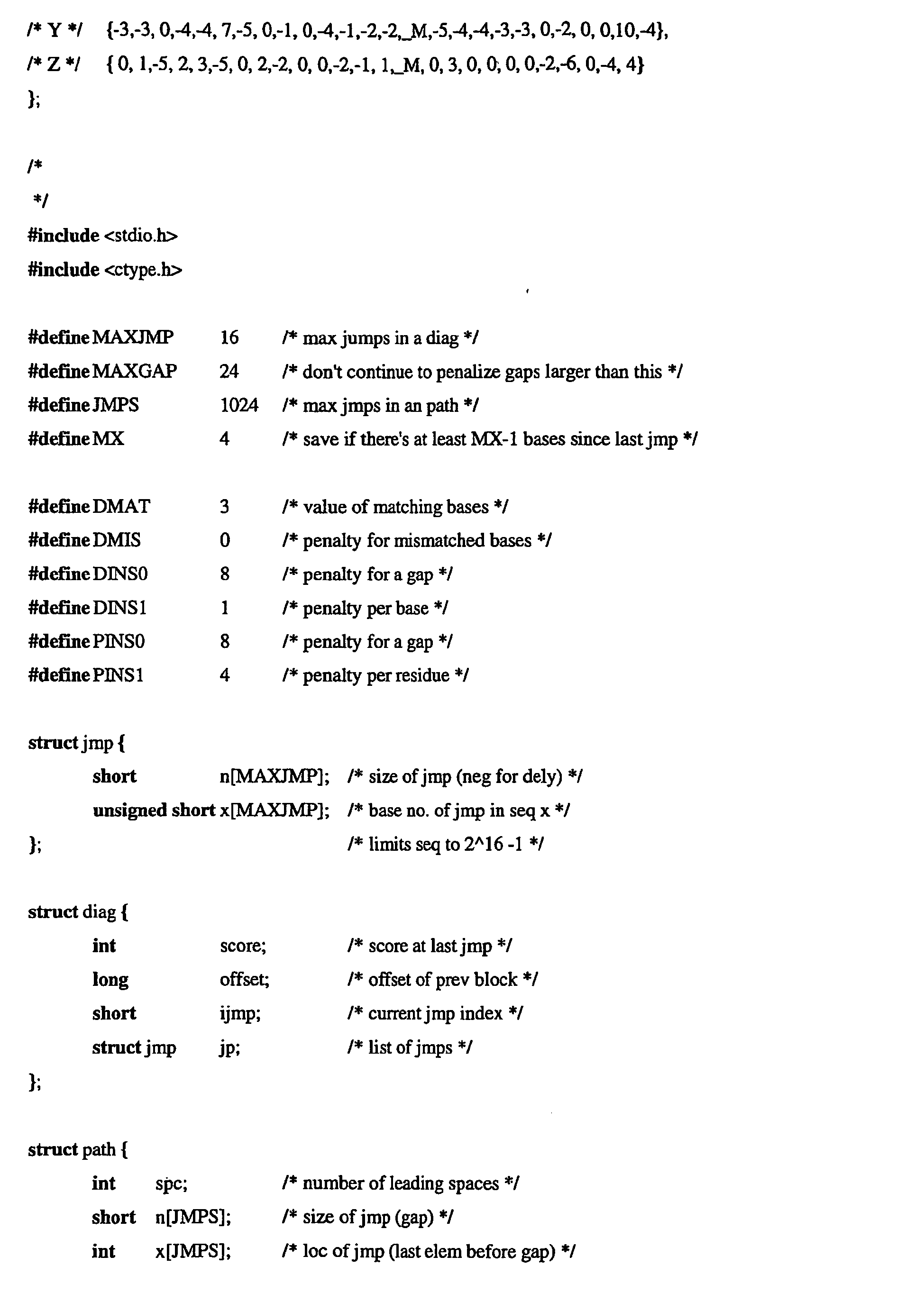

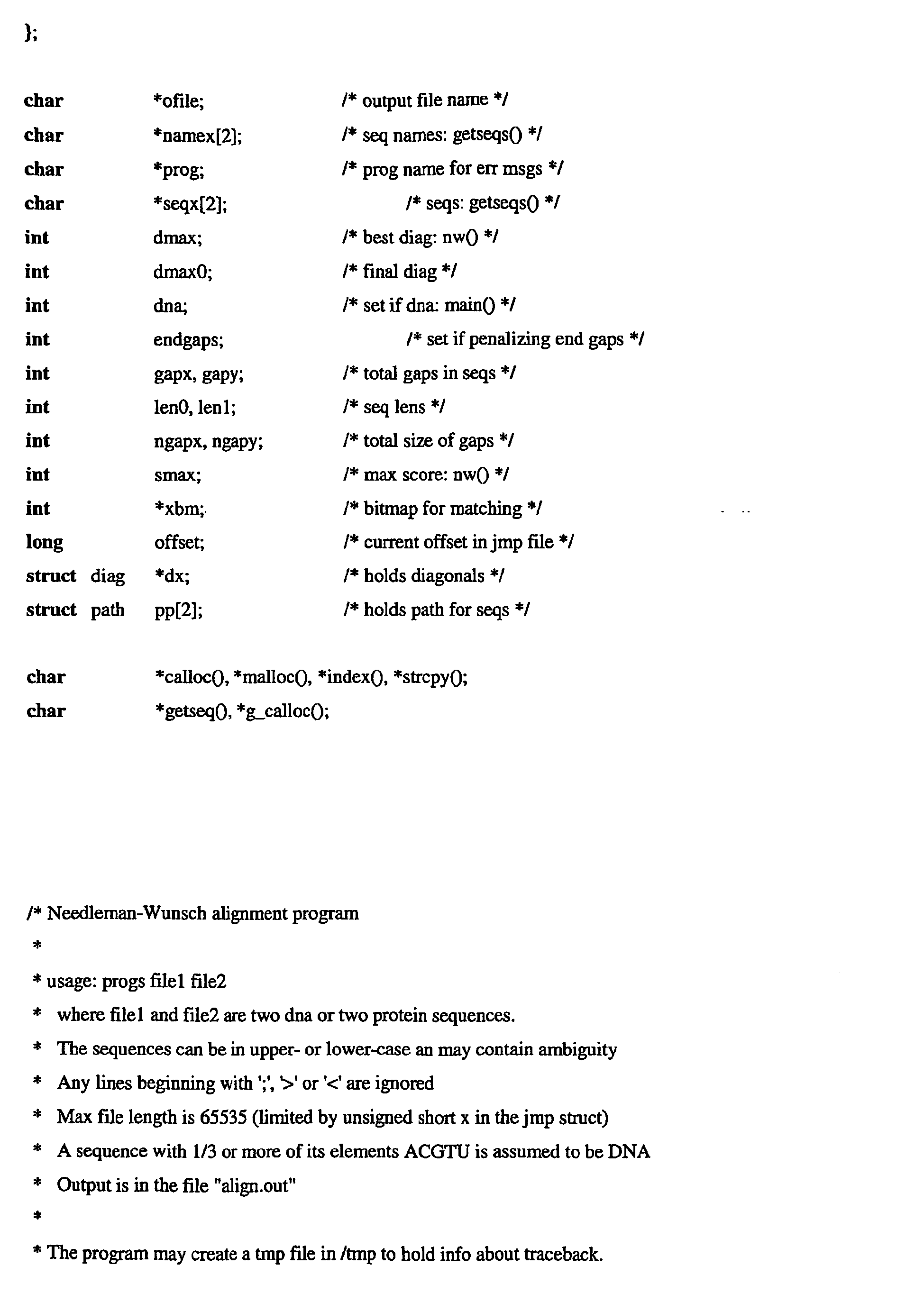

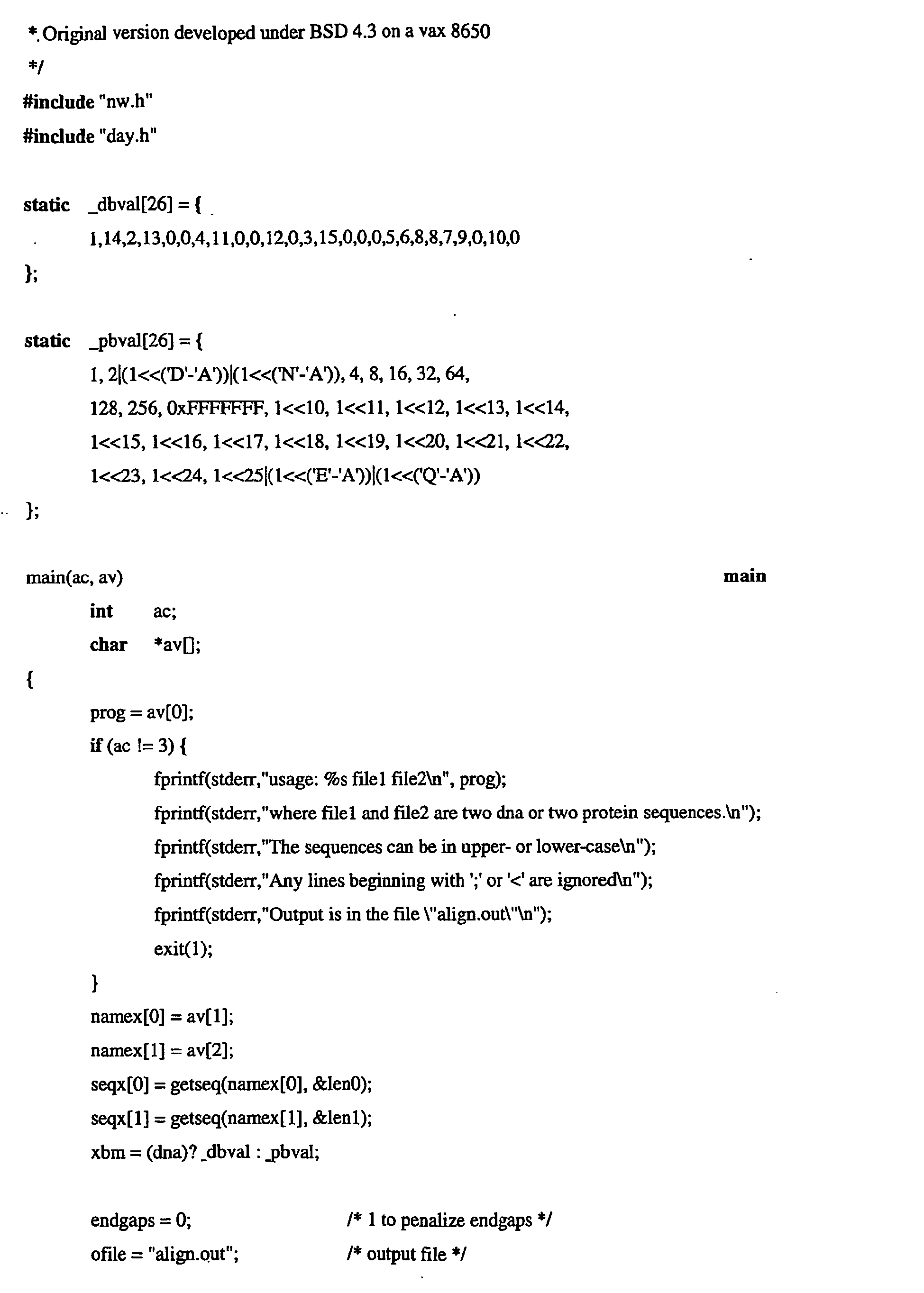

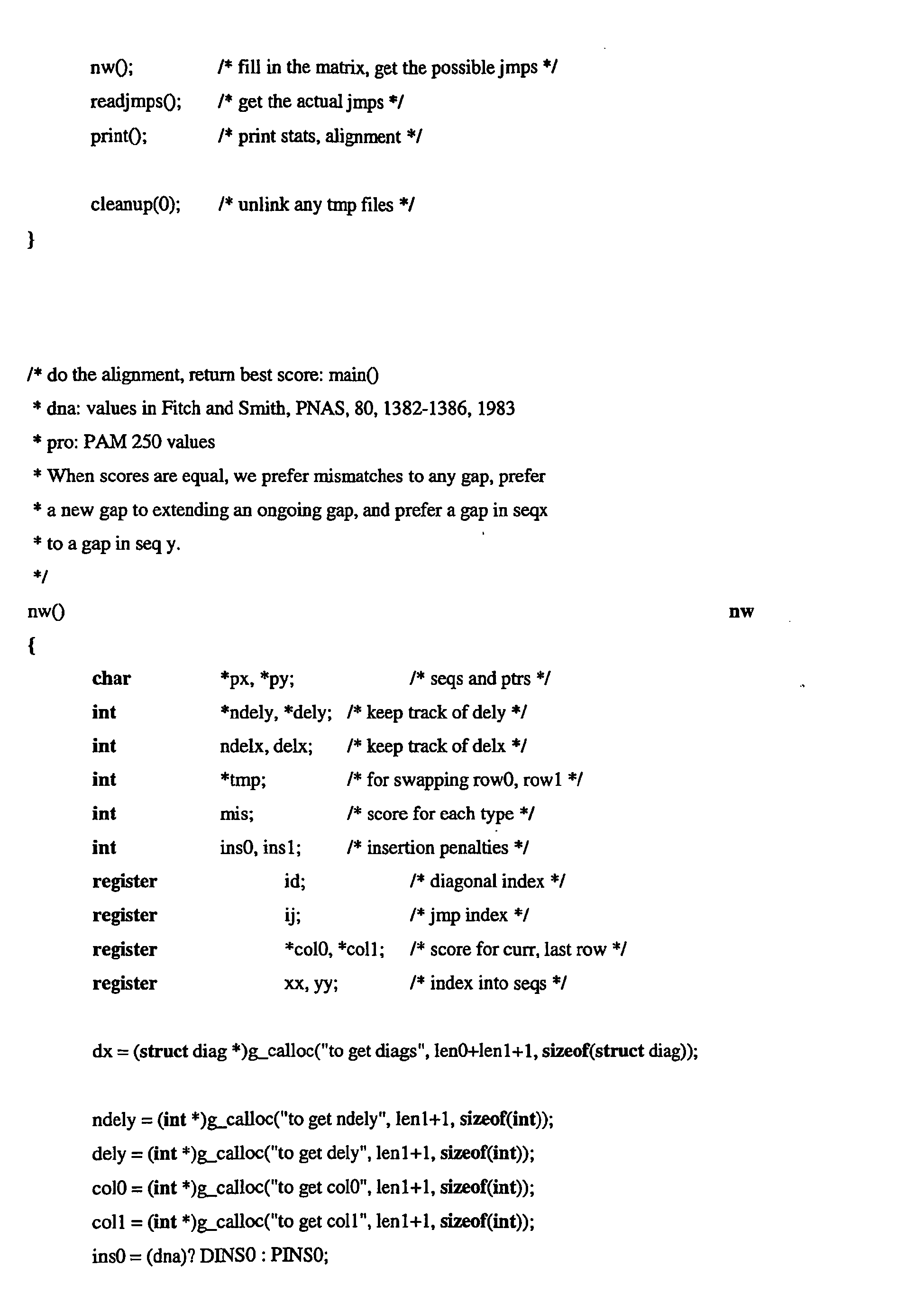

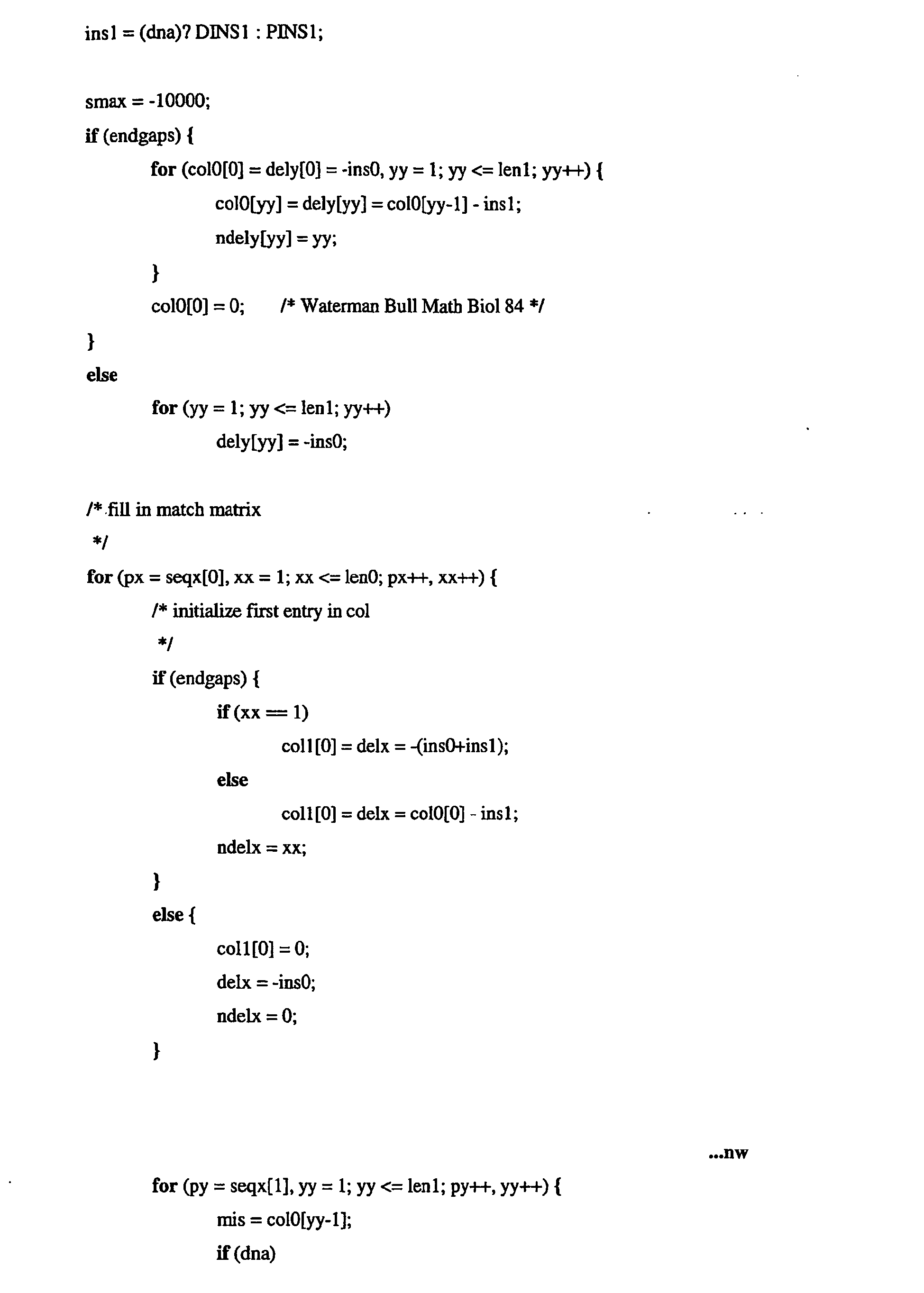

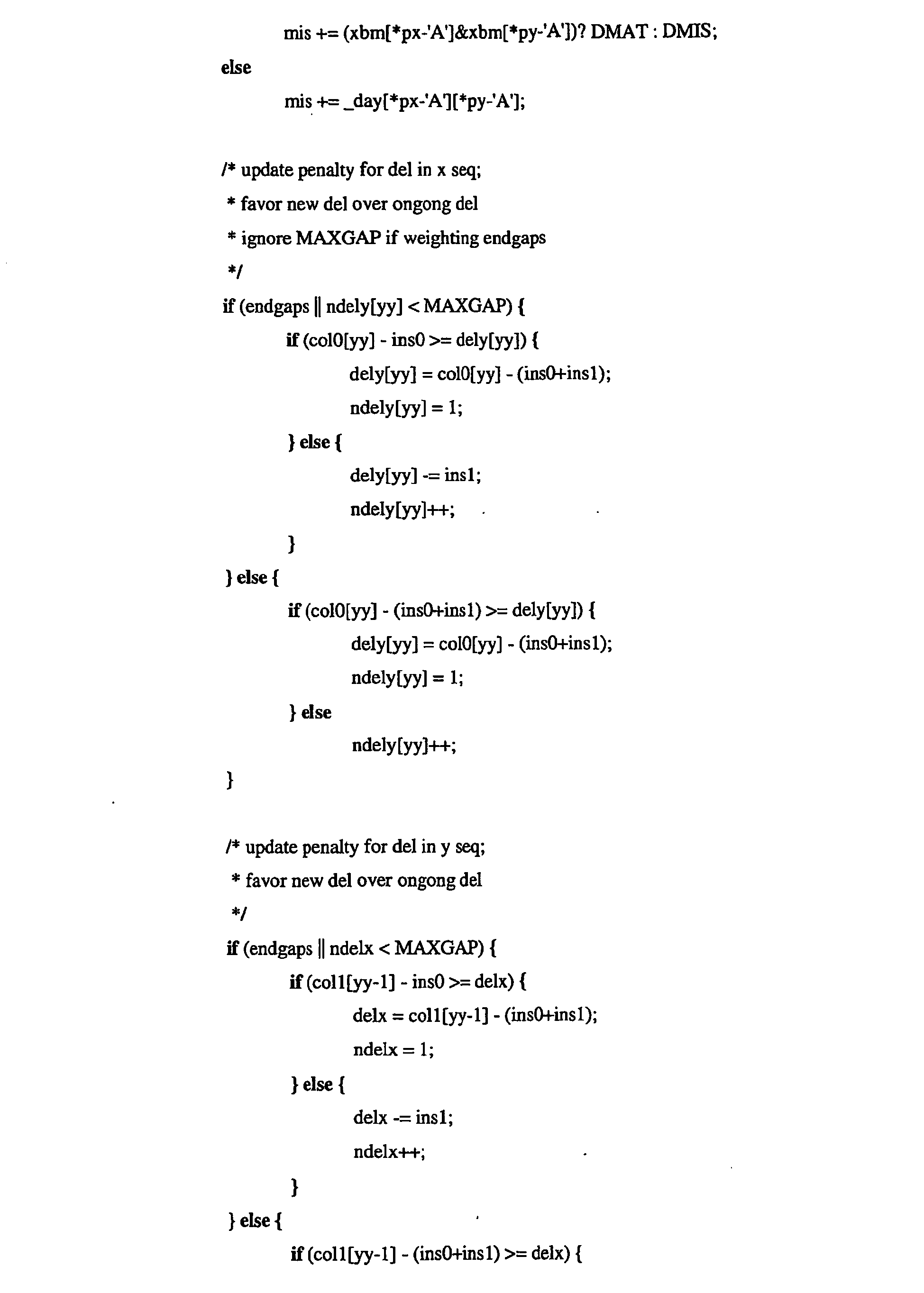

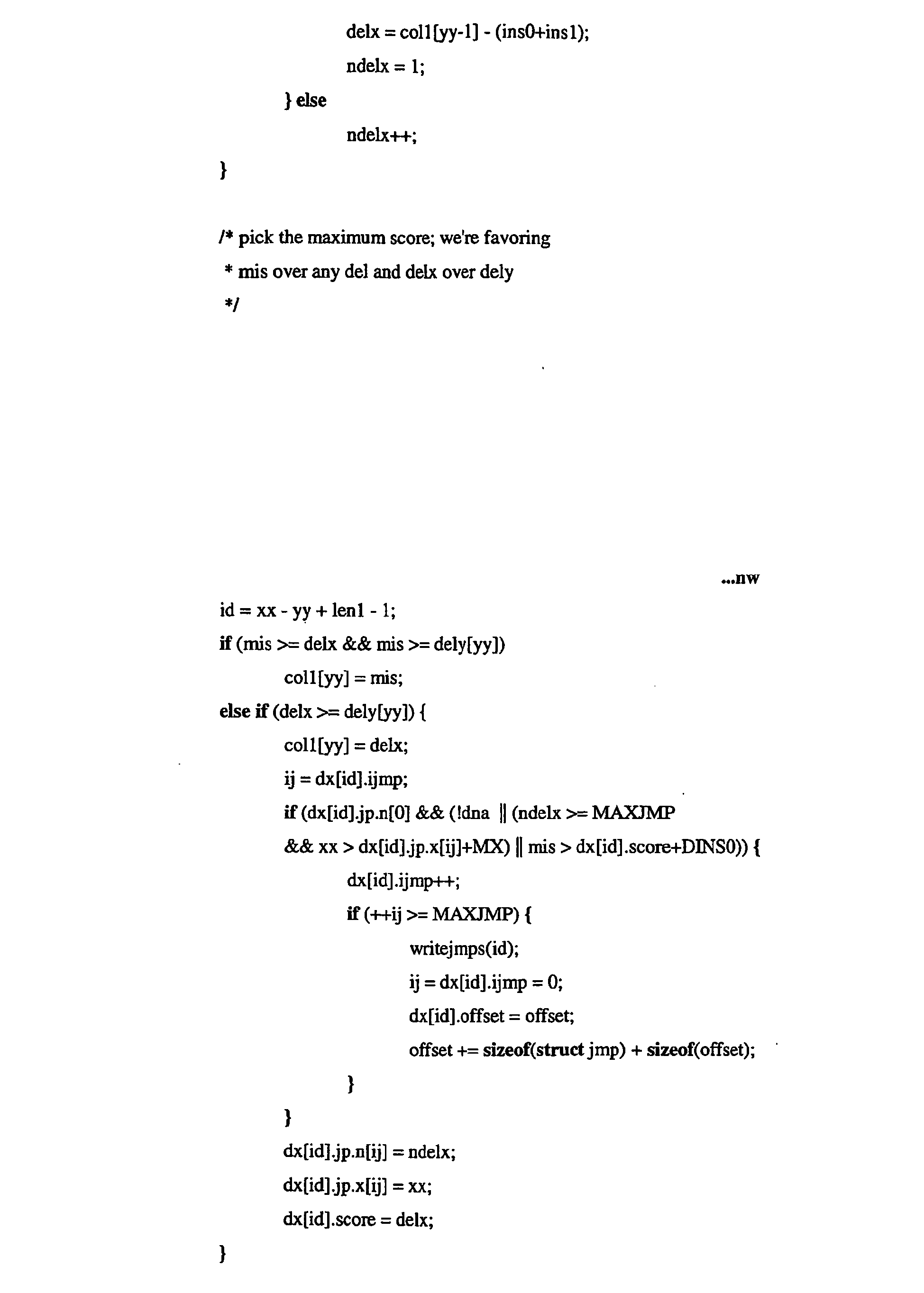

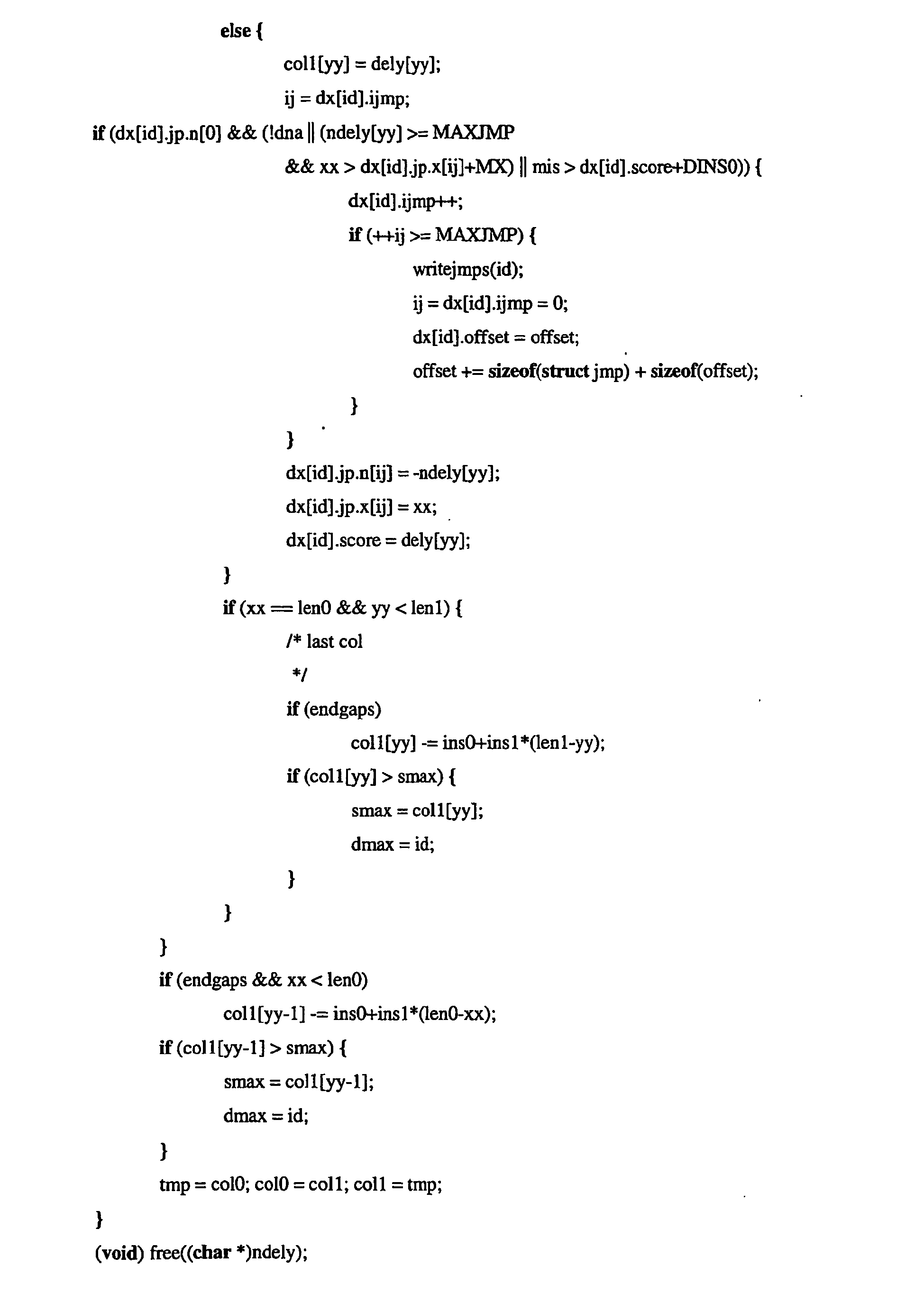

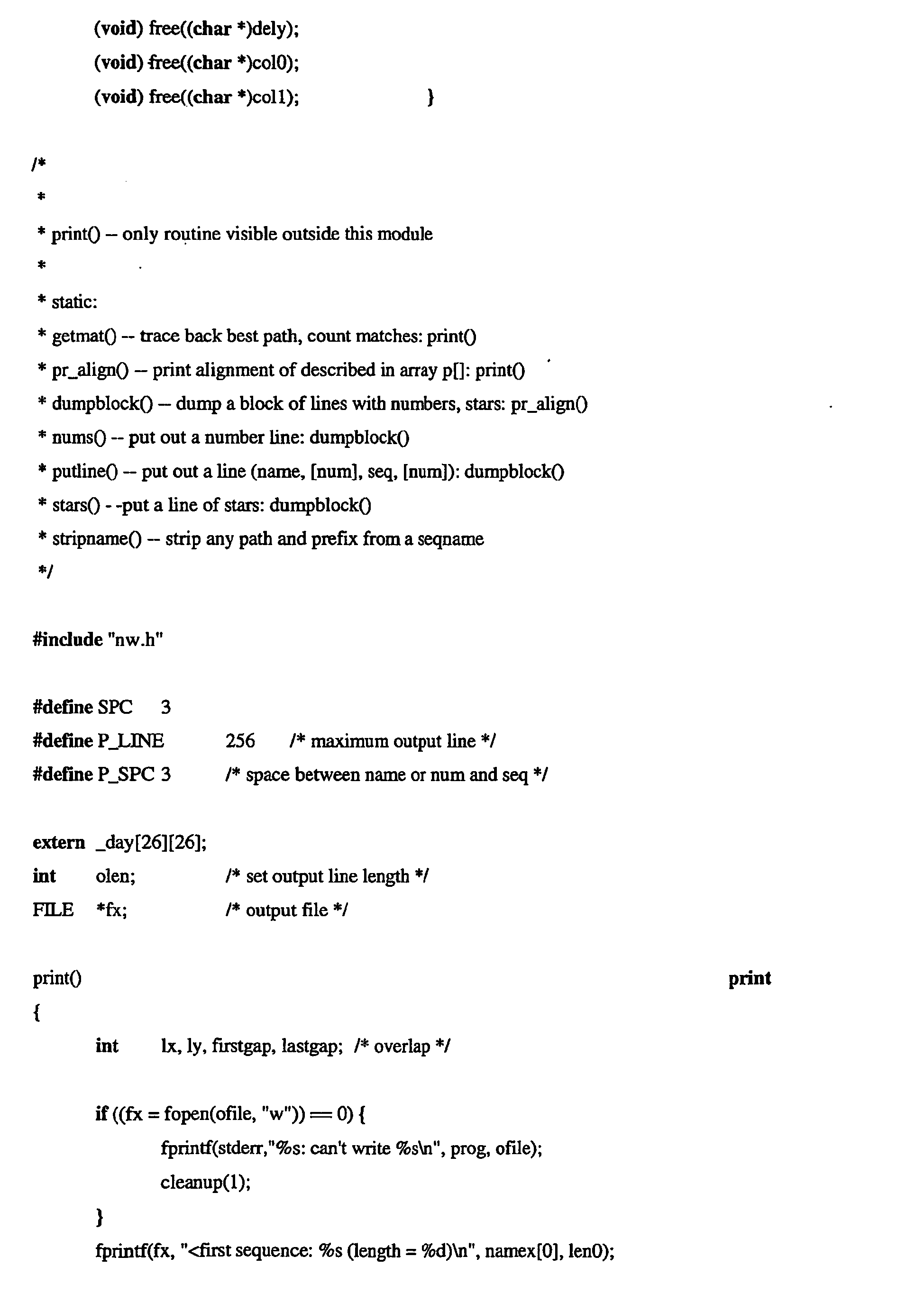

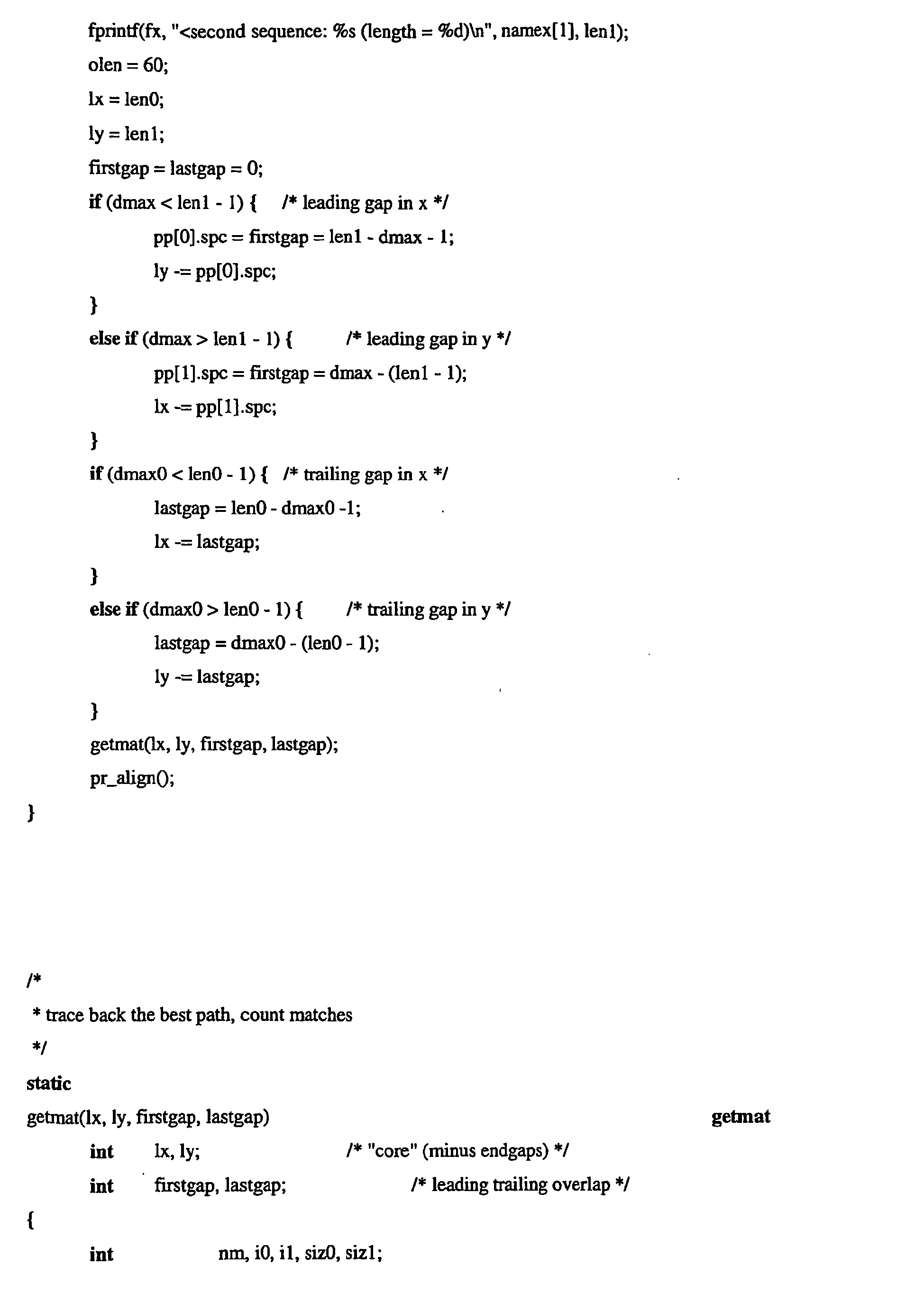

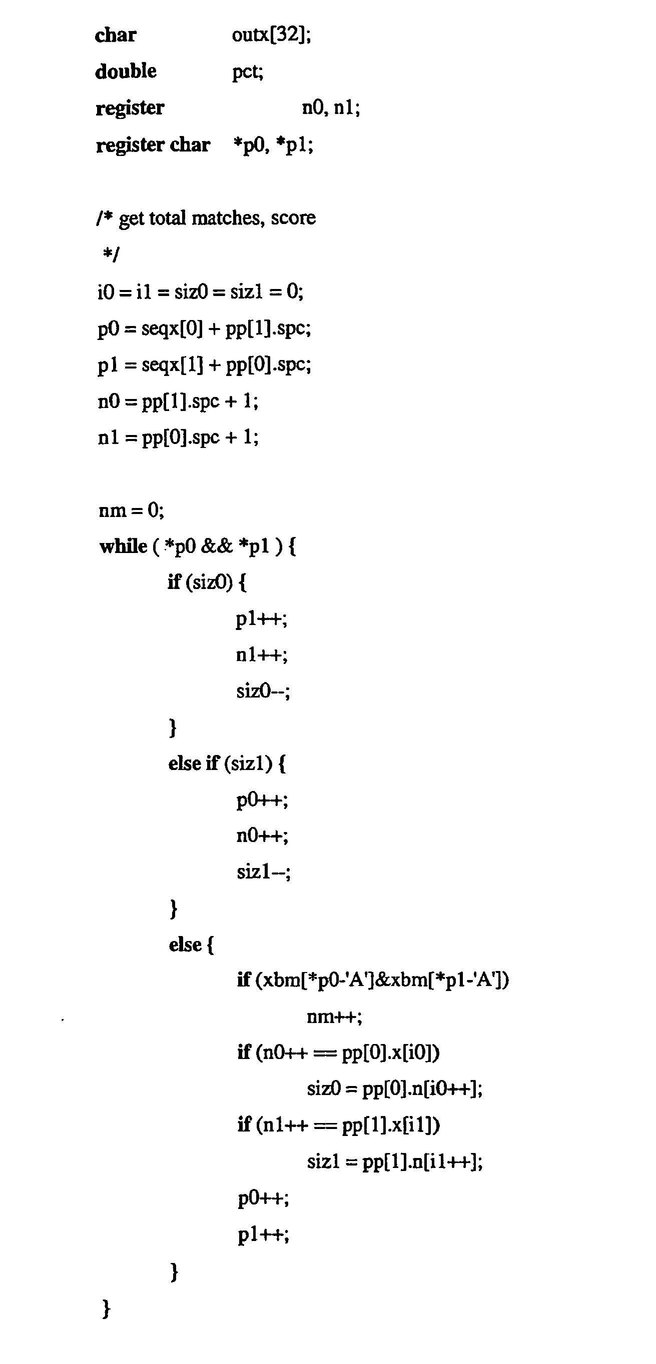

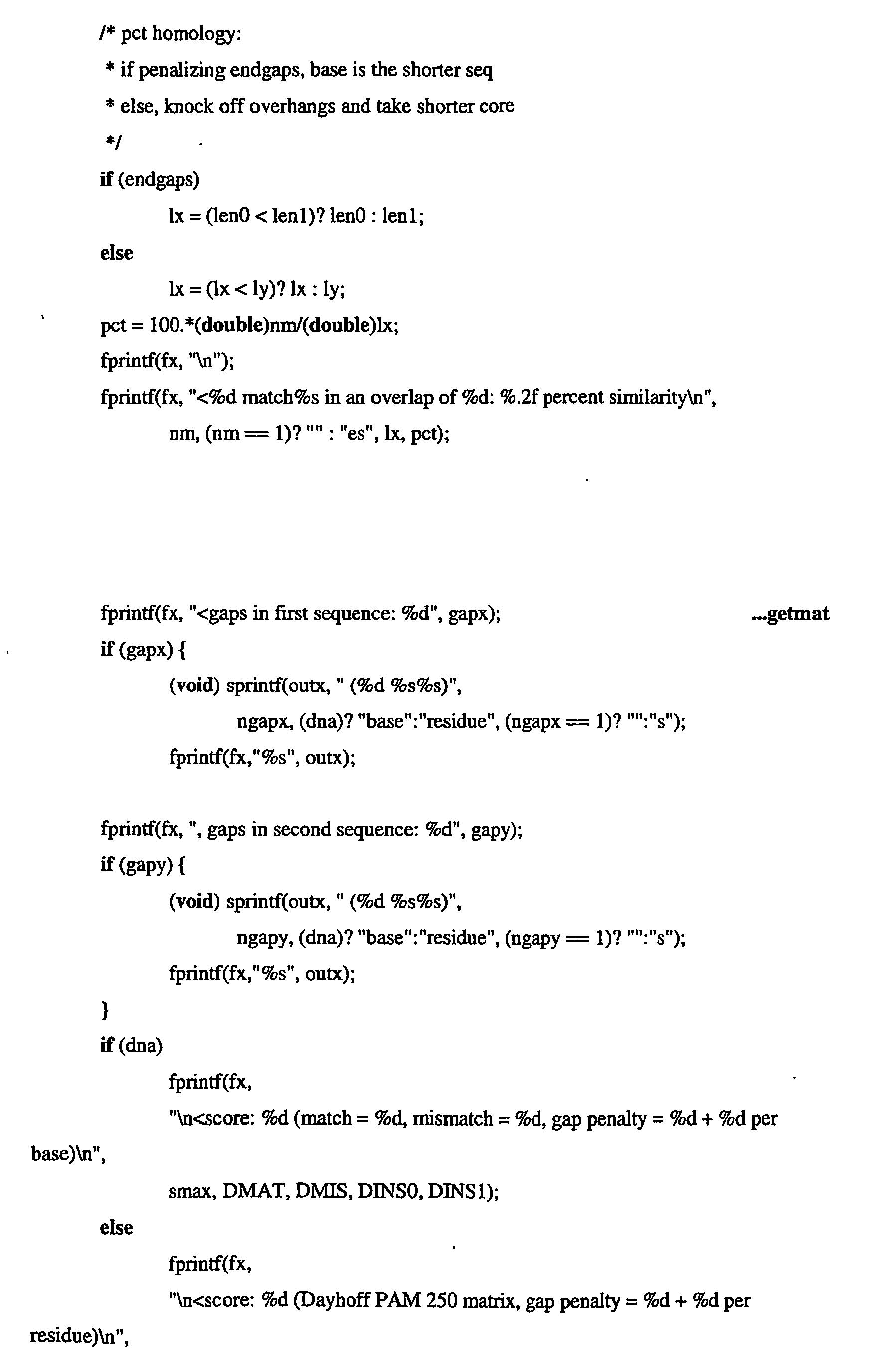

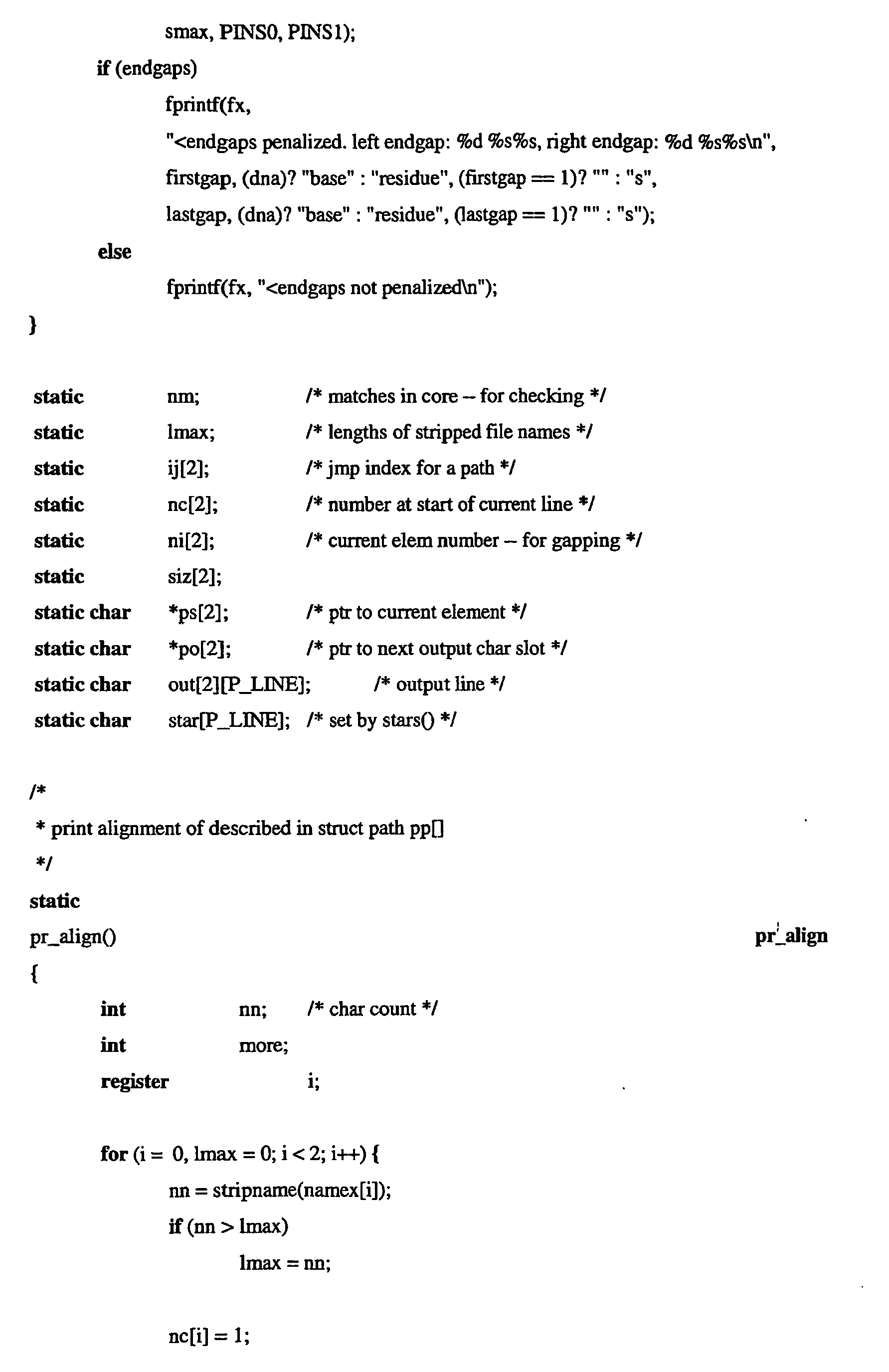

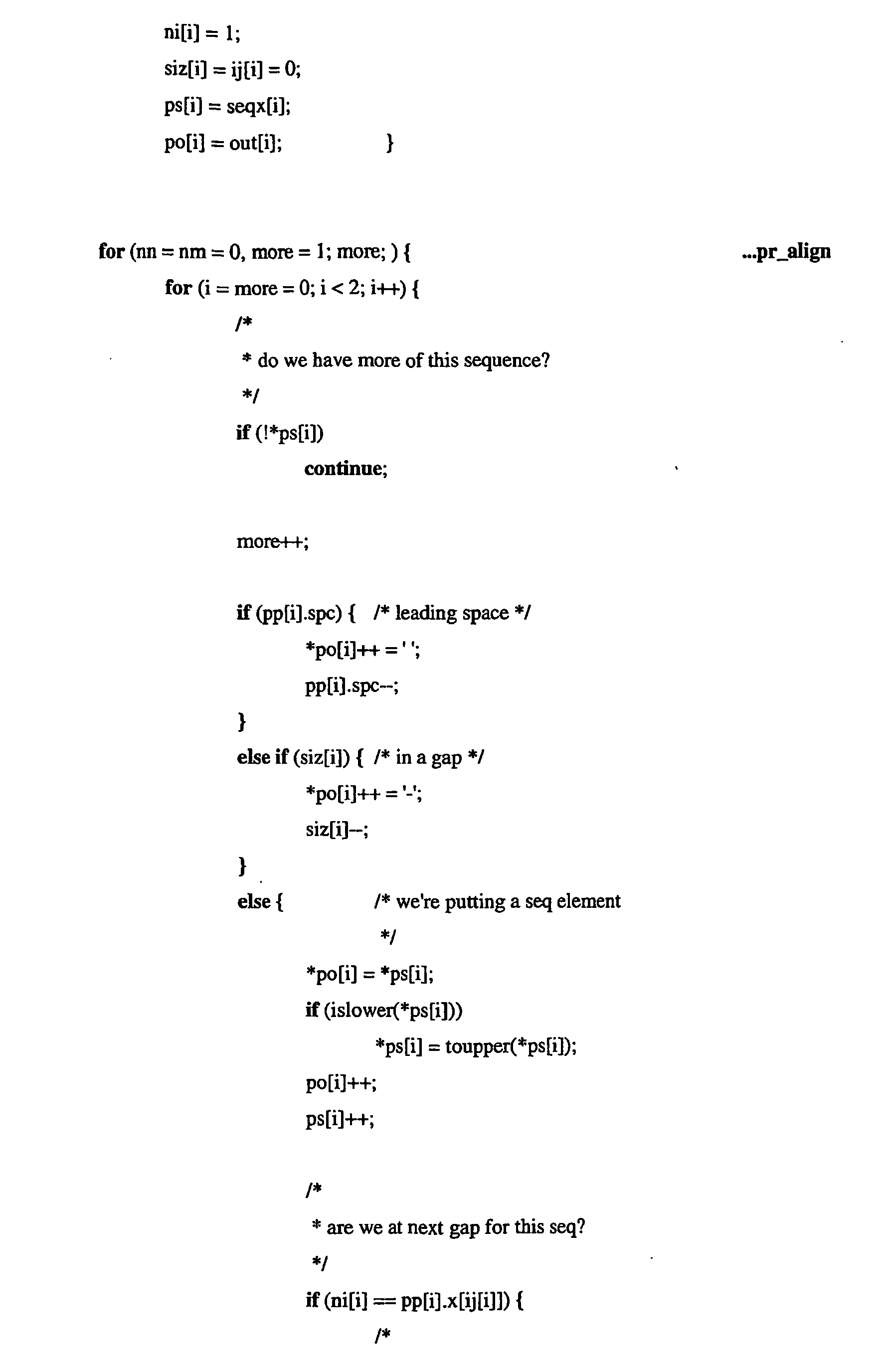

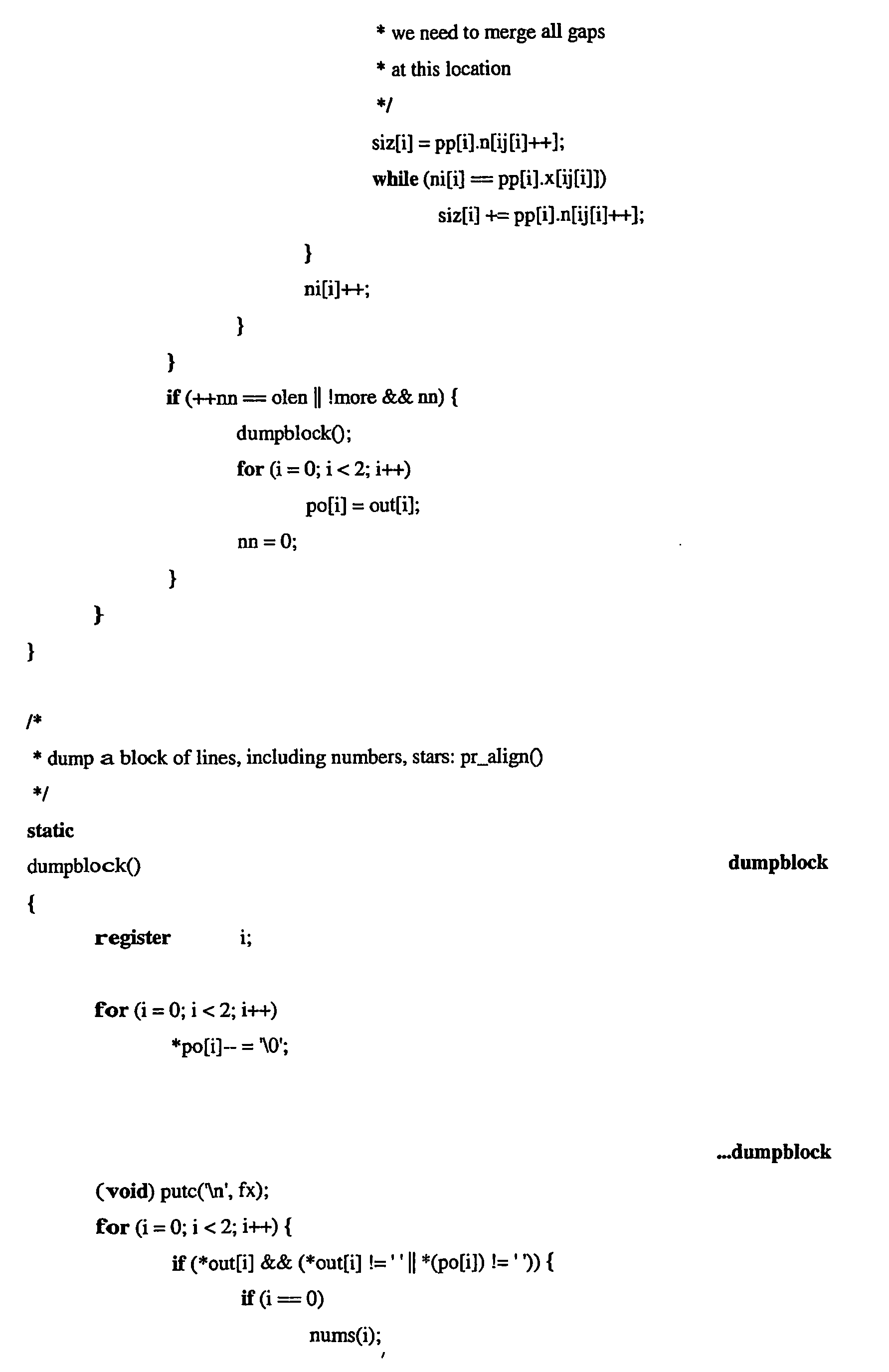

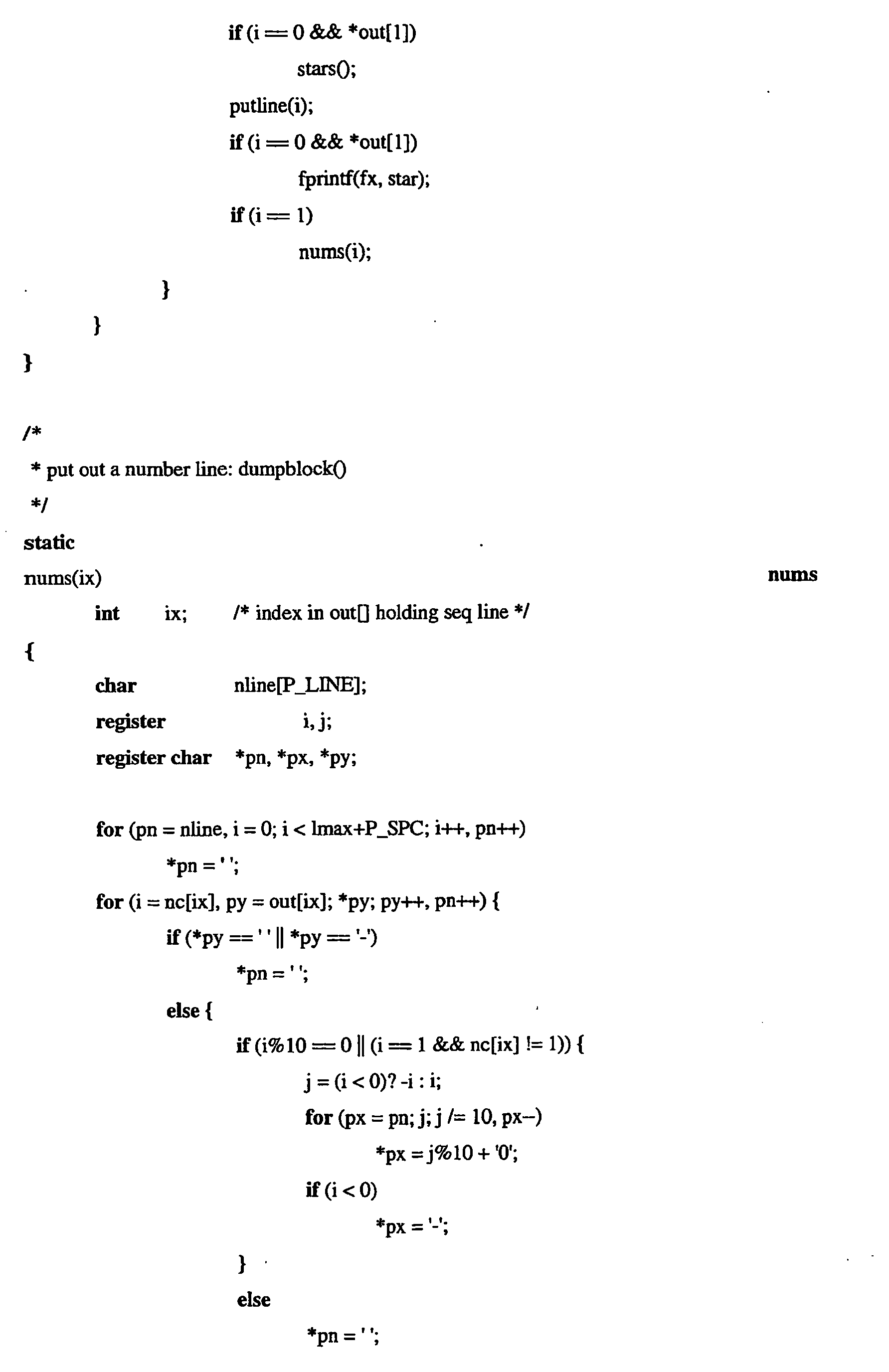

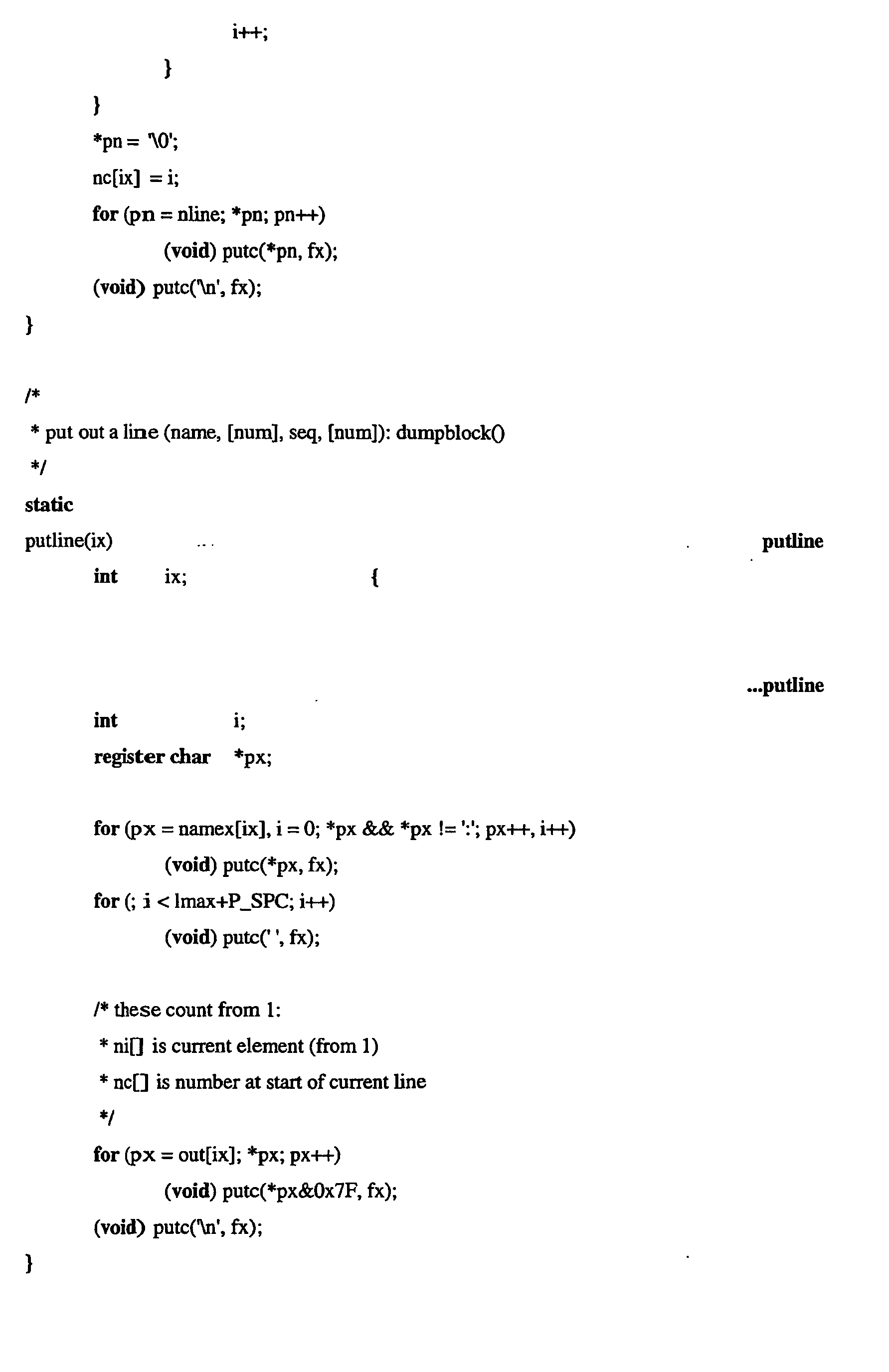

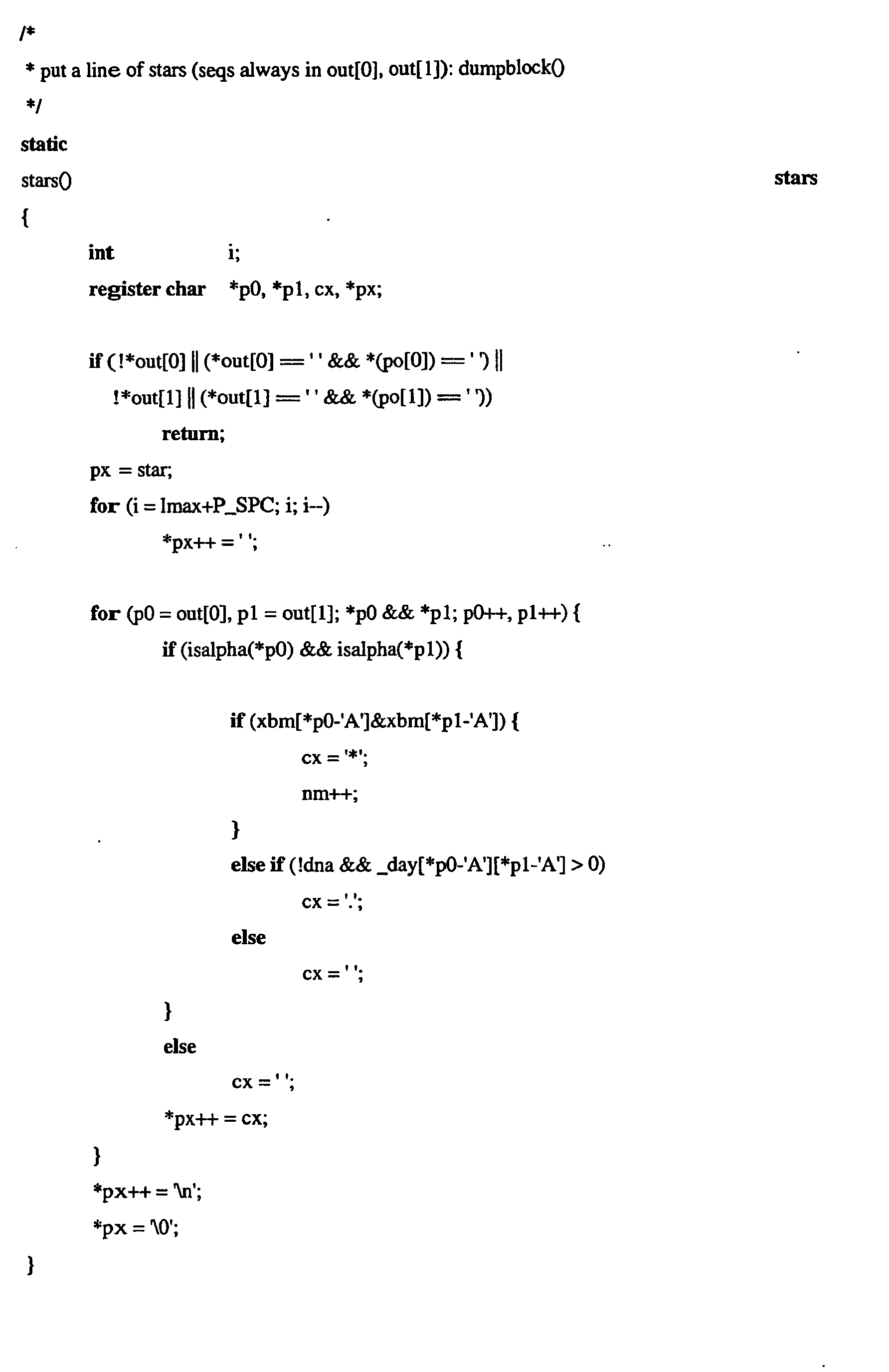

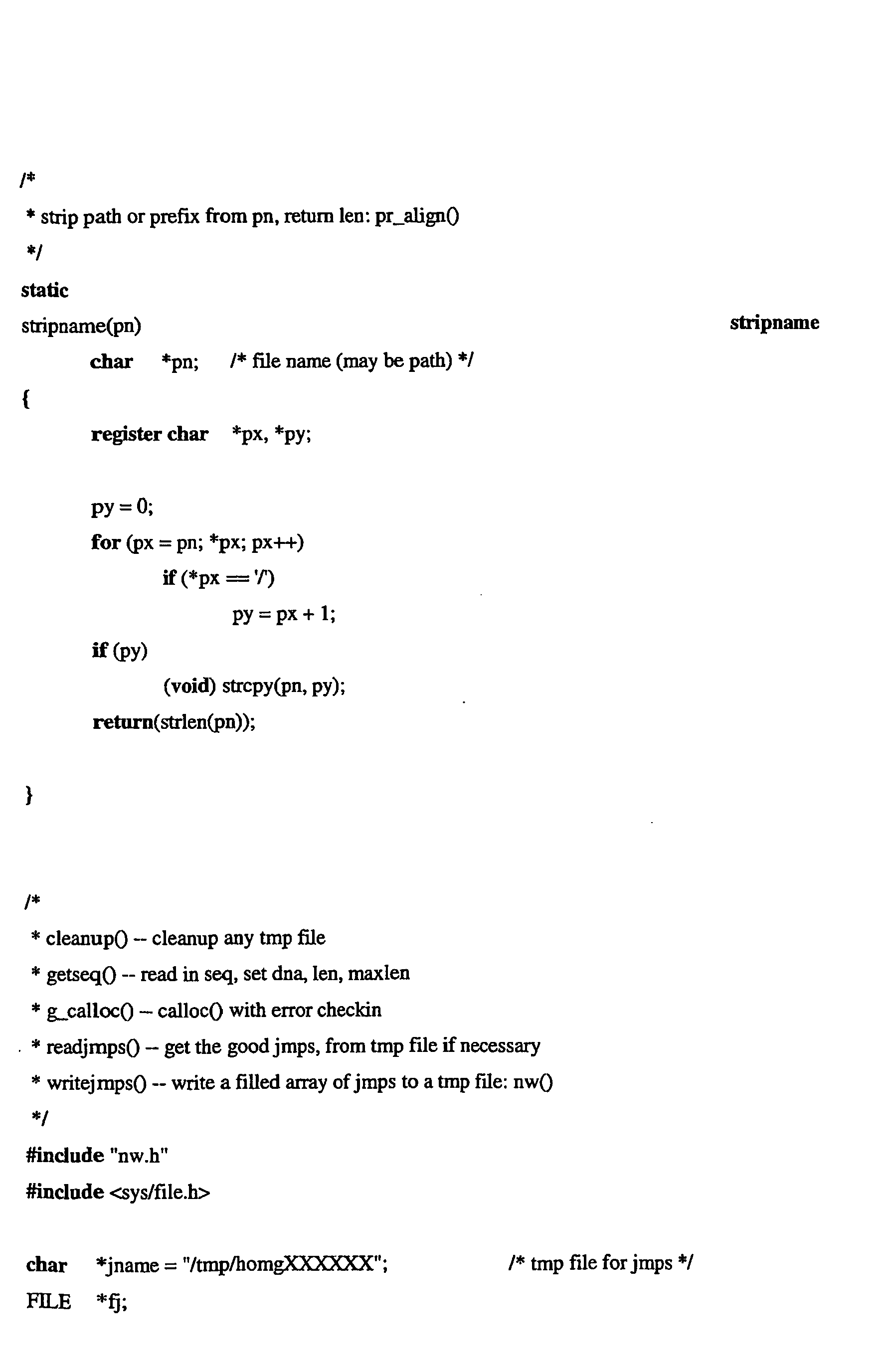

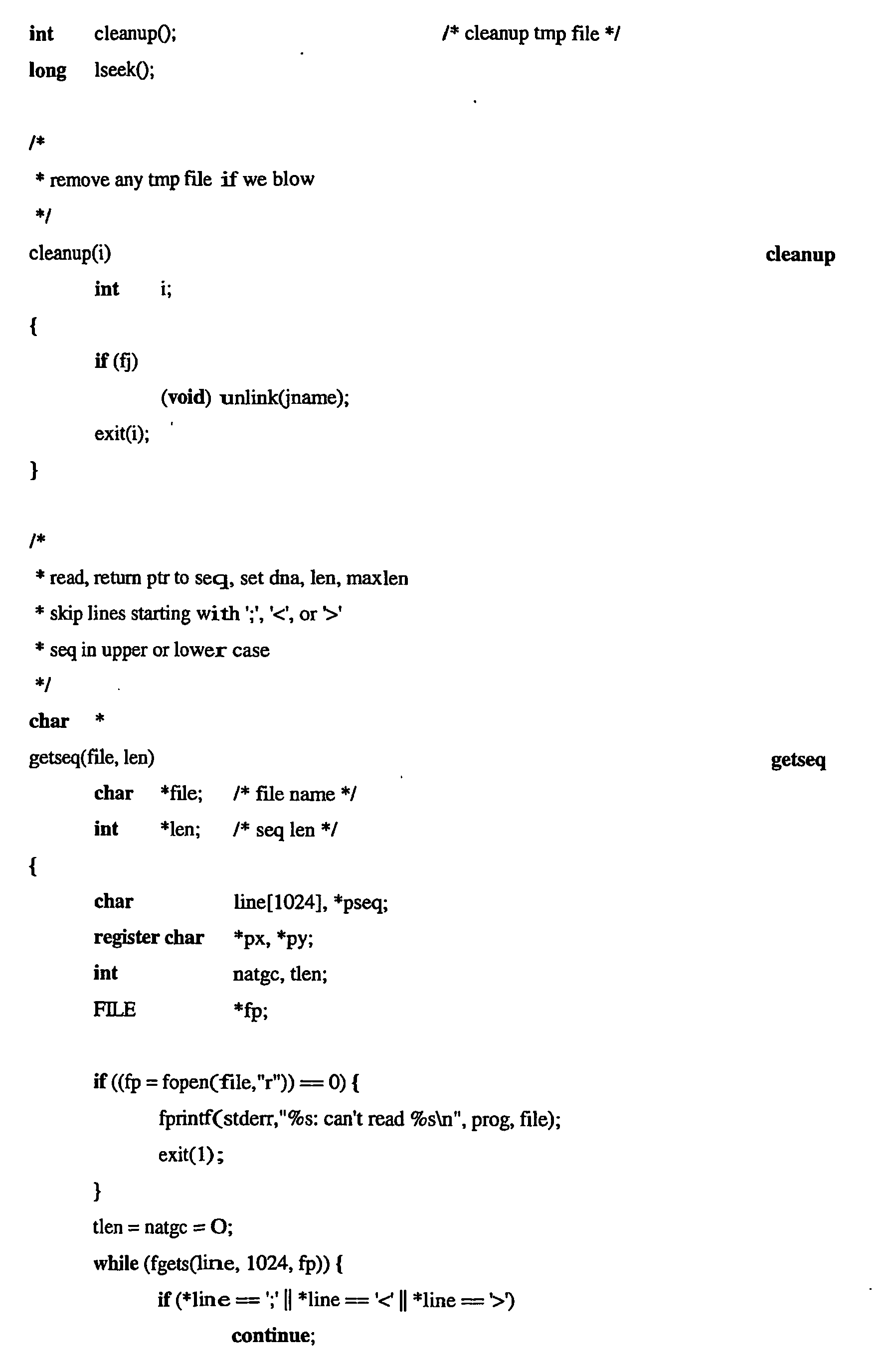

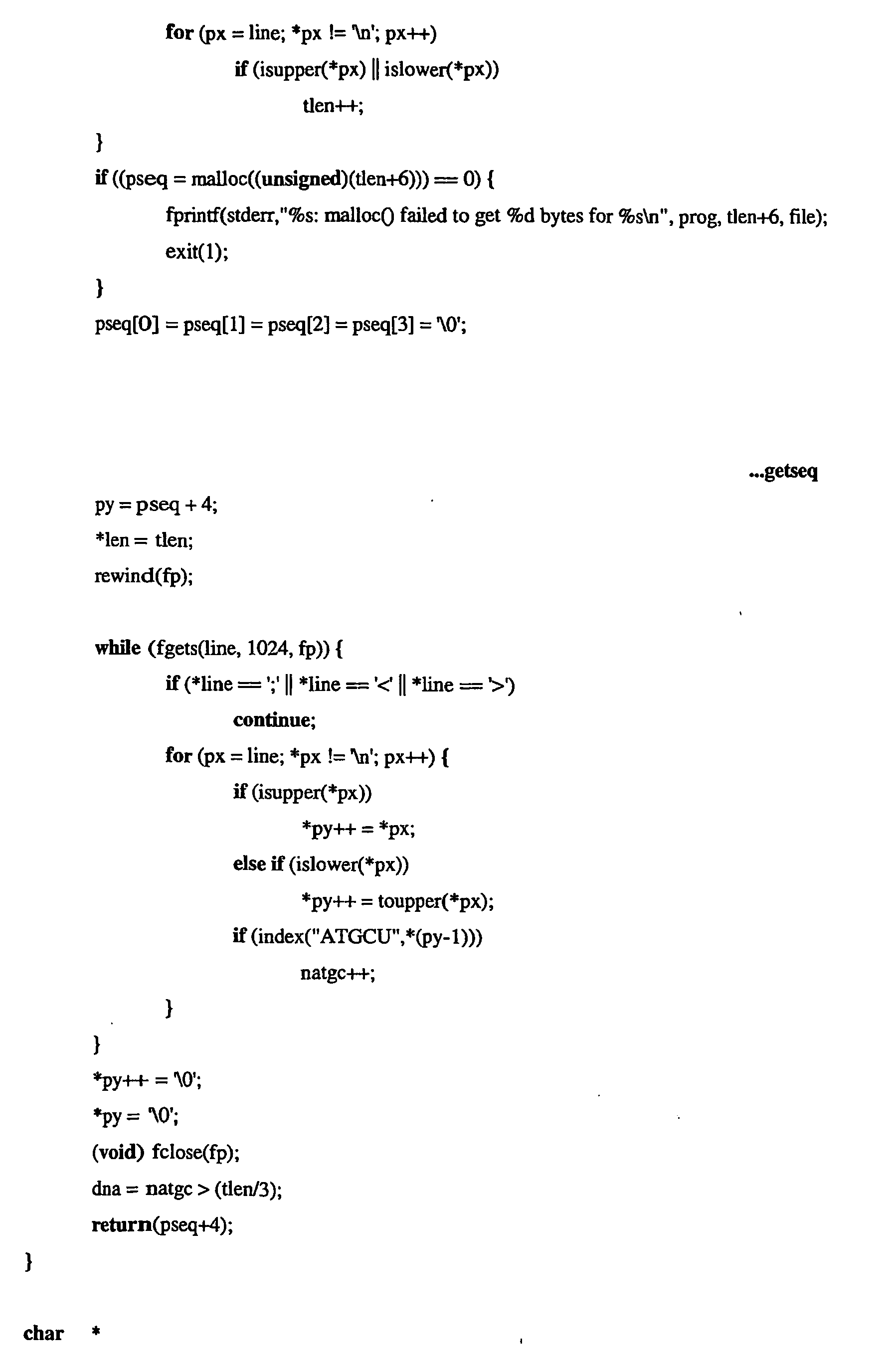

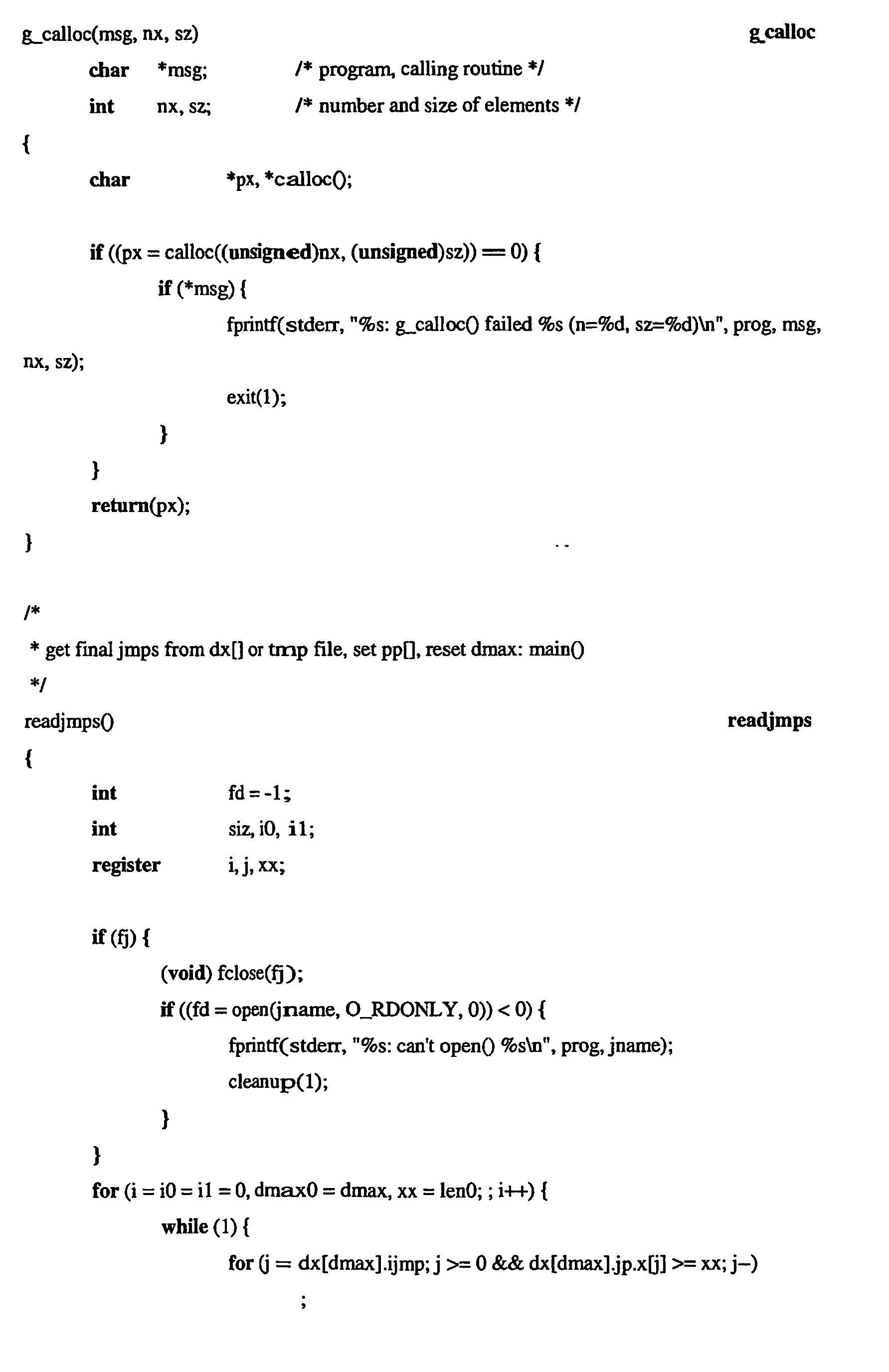

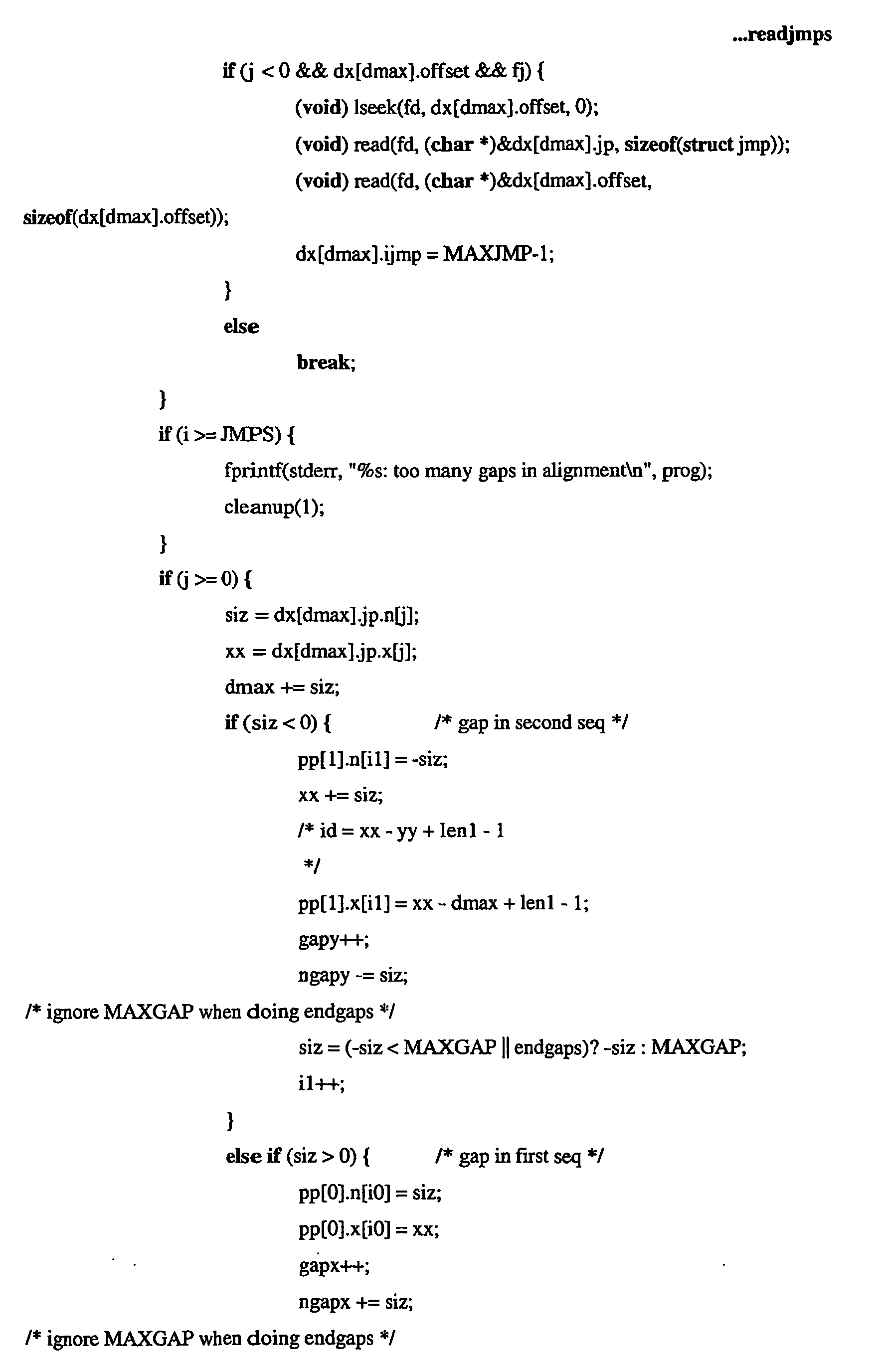

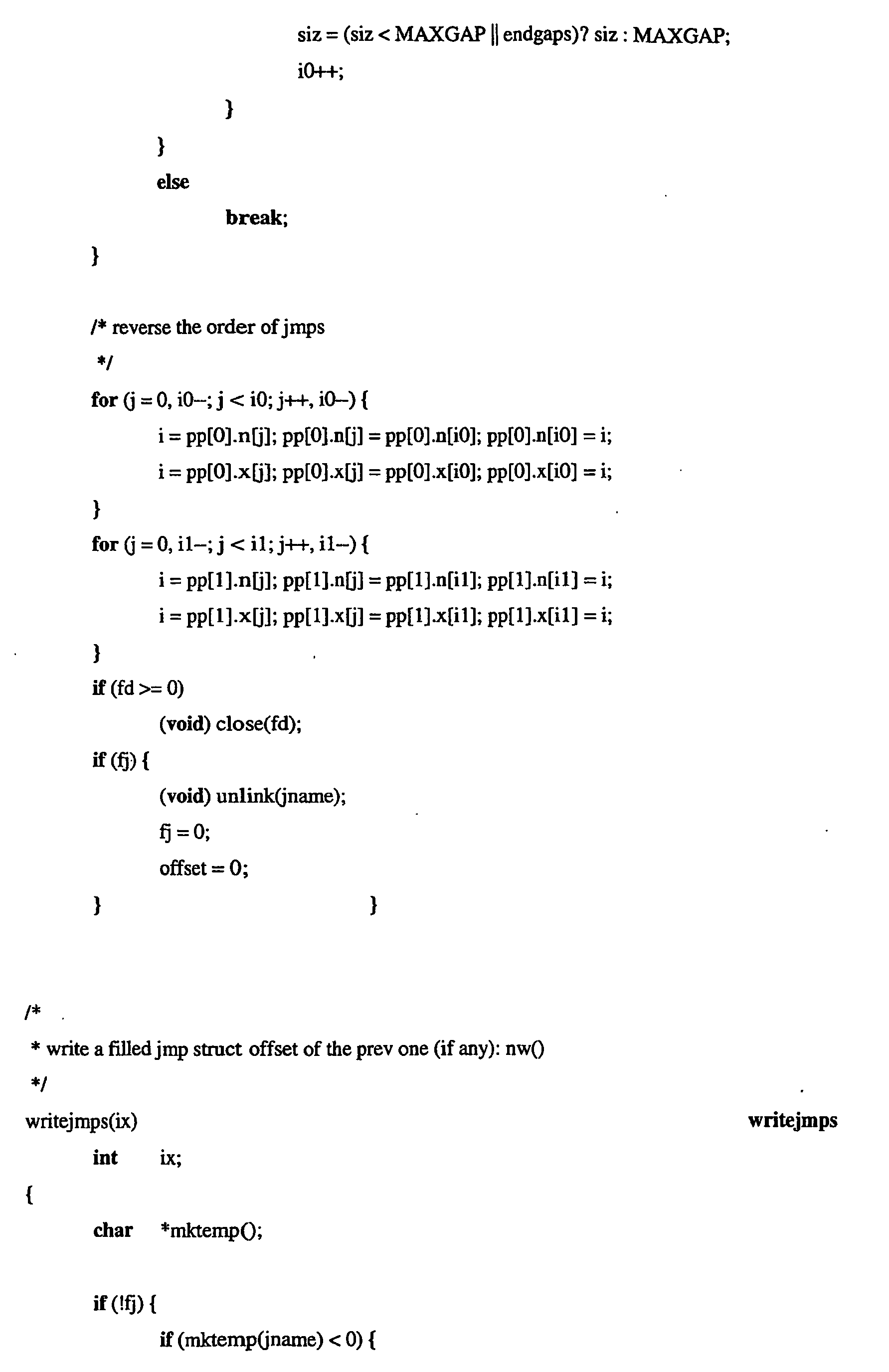

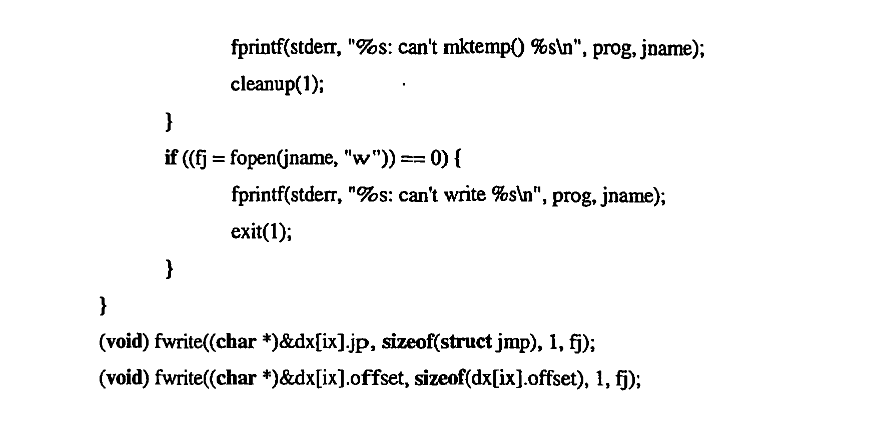

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, alpha.-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR.sub.2 ("amidate"), P(O)R, P(O)OR', CO or CH.sub.2 ("formacetal"), in which each R or R' is independently H or substituted or unsubstituted alkyl (1-20 C.) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "hepatocyte growth factor" or "HGF", as used herein, refers, unless specifically or contextually indicated otherwise, to any native or variant (whether native/naturally occurring or synthetic) HGF polypeptide that is capable of activating the HGF/c-met signaling pathway under conditions that permit such process to occur.

The terms "antibody" and "immunoglobulin" are used interchangeably in the broadest sense and include monoclonal antibodies (e.g., full length or intact monoclonal antibodies), polyclonal antibodies, monovalent antibodies, multivalent antibodies, multispecific antibodies (e.g., bispecific antibodies so long as they exhibit the desired biological activity) and antibody fragments. An antibody can be human, humanized and/or affinity matured.

"Antibody fragments" comprise only a portion of an intact antibody, wherein the portion preferably retains at least one, preferably most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e.g*., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least-one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol.1:105-115 (1998); Harris, Biochem. Soc. Transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more CDRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3.809-3813 (1994); Schier et al. Gene 169:147-155 (1995.); Yelton. et al. J. Immunol. 155:1994-2004 (1995); Jackson et al., J. Immunol. 154(7):3310-9 (1995); and Hawkins et al, J. Mol. BioL 226:889-896 (1992).

The term "antagonist" is used in the broadest sense herein, and includes any molecule that partially or fully blocks, inhibits, or neutralizes one or more of the biological activities of its target (e.g., c-met). For example, a "blocking" antibody or an "antagonist" antibody is one which inhibits or reduces biological activity of the antigen it binds (e.g., c-met).

A "disorder" is any condition that would benefit from treatment with a substance/molecule or method of the invention. This includes chronic and acute disorders or diseases including those pathological conditions which predispose the mammal to the disorder in question. Non-limiting examples of disorders to be treated herein include malignant and benign tumors; non-leukemias and lymphoid malignancies; neuronal, glial, astrocytal, hypothalamic and other glandular, macrophagal, epithelial, stromal and blastocoelic disorders; and inflammatory, immunologic and other angiogenesis-related disorders.

The terms "cancer" and "cancerous" refer to or describe the physiological condition in mammals that is typically characterized by unregulated cell growth/proliferation. Examples of cancer include but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

An "autoimmune disease" herein is a non-malignant disease or disorder arising from and directed against an individual's own tissues. The autoimmune diseases herein specifically exclude malignant or cancerous diseases or conditions, especially excluding B cell lymphoma, acute lymphoblastic leukemia (ALL), chronic lymphocytic leukemia (CLL), Hairy cell leukemia and chronic myeloblastic leukemia. Examples of autoimmune diseases or disorders include, but are not limited to, inflammatory responses such as inflammatory skin diseases including psoriasis and dermatitis (e.g. atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (e.g. Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia) ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antagonists of the invention are used to delay development of a disease or disorder.

An "effective amount" refers to an amount effective, at dosages and for periods of time necessary, to achieve the desired therapeutic or prophylactic result. A "therapeutically effective amount" of an antagonist of the invention may vary according to factors such as the disease state, age, sex, and weight of the individual, and the ability of the antagonist to elicit a desired response in the individual. A therapeutically effective amount is also one in which any toxic or detrimental effects of the antagonist are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

### Vector Construction

Polynucleotide sequences encoding the polypeptides described herein can be obtained using standard recombinant techniques. Desired polynucleotide sequences may be isolated and sequenced from appropriate source cells. Source cells for antibodies would include antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in a host cell. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication (in particular when the vector is inserted into a prokaryotic cell), a selection marker gene, a promoter, a ribosome binding site (RBS), a signal sequence, the heterologous nucleic acid insert and a transcription termination sequence.

In general, plasmid vectors containing replicon and control sequences which are derived from a species compatible with the host cell are used in connection with these hosts. The vector ordinarily carries a replication site, as well as marking sequences which are capable of providing phenotypic selection in transformed cells. For example, E. *coli* is typically transformed using pBR322, a plasmid derived from an E. *coli* species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins.

In addition, phage vectors containing replicon and control sequences that are compatible with the host microorganism can be used as transforming vectors in connection with these hosts. For example, bacteriophage such as λGEM.TM.-11 may be utilized in making a recombinant vector which can be used to transform susceptible host cells such as *E. coli* LE392.

Either constitutive or inducible promoters can be used in the present invention, in accordance with the needs of a particular situation, which can be ascertained by one skilled in the art. A large number of promoters recognized by a variety of potential host cells are well known. The selected promoter can be operably linked to cistron DNA encoding a polypeptide described herein by removing the promoter from the source DNA via restriction enzyme digestion and inserting the isolated promoter sequence into the vector of choice. Both the native promoter sequence and many heterologous promoters may be used to direct amplification and/or expression of the target genes. However, heterologous promoters are preferred, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the *tac* or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

In some embodiments, each cistron within a recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PeIB, OmpA and MBP.

Prokaryotic host cells suitable for expressing polypeptides include Archaebacteria and Eubacteria, such as Gram-negative or Gram-positive organisms. Examples of useful bacteria include Escherichia (e.g., *E. coli*), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. Preferably, gram-negative cells are used. Preferably the host cell should secrete minimal amounts of proteolytic enzymes, and additional protease inhibitors may desirably be incorporated in the cell culture.

### Polypeptide Production

Host cells are transformed or transfected with the above-described expression vectors and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ precipitation and electroporation. Successful transfection is generally recognized when any indication of the operation of this vector occurs within the host cell.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic host cells can be grown in any media known in the art to be suitable for culture of the selected host cells. Examples of suitable media include luria broth (LB) plus necessary nutrient supplements. The media may also contain a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For E. *coli* growth, for example, the preferred temperature ranges from about 20°C to about 39°C, more preferably from about 25°C to about 37°C, even more preferably at about 30°C. The pH of the medium may be any pH ranging from about 5 to about 9, depending mainly on the host organism. For E. *coli,* the pH is preferably from about 6.8 to about 7.4, and more preferably about 7.0.

If an inducible promoter is used in the expression vector, protein expression is induced under conditions suitable for the activation of the promoter. For example, if a PhoA promoter is used for controlling transcription, the transformed host cells may be cultured in a phosphate-limiting medium for induction. A variety of other inducers may be used, according to the vector construct employed, as is known in the art.

Polypeptides expressed in a microorganism may be secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therefrom. Cells may be removed from the culture and the culture supernatant being filtered and concentrated for further purification of the proteins produced. The expressed polypeptides can be further isolated and identified using commonly known methods such as fractionation on immunoaffinity or ion-exchange columns; ethanol precipitation; reverse phase HPLC; chromatography on silica or on a cation exchange resin such as DEAE; chromatofocusing; SDS-PAGE; ammonium sulfate precipitation; gel filtration using, for example, Sephadex G-75; hydrophobic affinity resins, ligand affinity using a suitable antigen immobilized on a matrix and Western blot assay.

Besides prokaryotic host cells, eukaryotic host cell systems are also well established in the art. See, e.g., U.S. Pat. Nos. 4,816,567. Suitable hosts include mammalian cell lines such as CHO, and insect cells such as those described below.

### Polypeptide Purification

Polypeptides that are produced may be purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75.

### Methods Of The Invention

The invention provides various methods based on the identification of domains within c-met that are important for activation of the HGF/c-met signaling pathway. It is demonstrated herein that interference with such domains modulates HGF/c-met activities.

Various substances or molecules (including peptides/polypeptides, antibodies, etc.) may be employed as therapeutic agents. These substances or molecules can be formulated according to known methods to prepare pharmaceutically useful compositions, whereby the product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™ or PEG.

The formulations to be used for in *vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When *in vivo* administration of an antagonist of the invention is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a substance or molecule is desired in a formulation with release characteristics suitable for the treatment of any disease or disorder requiring administration of the substance or molecule, microencapsulation of the substance or molecule is contemplated. Microencapsulation of recombinant proteins for sustained release has been successfully performed with human growth hormone (rhGH], interferon- (rhIFN-), interleukin-2, and MN rgp120. Johnson et al., Nat. Med., 2:795-799 (1996); Yasuda, Biomed. Ther. 27:1221-1223 (1993); Hora et al., Bio/Technology. 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

This invention encompasses methods of screening compounds to identify those that inhibit HGF/c-met signaling through interfering with c-met Sema domain function(s) (e.g., c-met dimerization, ligand binding). Screening assays are designed to identify compounds that bind or complex with c-met Sema sequence, or otherwise interfere with the interaction of c-met Sema domain with other cellular proteins. Such screening assays will include assays amenable to high-throughput screening of chemical/compound libraries, making them particularly suitable for identifying drug candidates (e.g., peptides/polypeptides, small molecules, antibodies, etc.).

The assays can be performed in a variety of formats, including protein-protein binding assays, biochemical screening assays, immunoassays, and cell-based assays, which are well characterized in the art.

All assays for antagonists are common in that they call for contacting the drug candidate with c-met Sema domain under conditions and for a time sufficient to allow c-met (Sema domain) to interact with its binding partner.

In binding assays, the interaction is binding and the complex formed can be isolated or detected in the reaction mixture. In a particular embodiment, a candidate substance or molecule is immobilized on a solid phase, e.g., on a microtiter plate, by covalent or non-covalent attachments. Non-covalent attachment generally is accomplished by coating the solid surface with a solution of the substance/molecule and drying. Alternatively, an immobilized affinity molecule, such as an antibody, e.g., a monoclonal antibody, specific for the substance/molecule to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to c-met Sema domain, its interaction with the domain can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of c-met Sema domain with its partner can be tested as follows: usually a reaction mixture is prepared containing the c-met Sema sequence, or portion thereof, and a binding partner (e.g., another polypeptide comprising Sema, such as the extracellular domain of c-met) under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and c-met Sema sequence present in the mixture is monitored as described hereinabove. The formation of a complex in the control reaction(s) but not in the reaction mixture containing the test compound indicates that the test compound interferes with the interaction of c-met Sema domain and its binding partner.

To assay for inhibitors (such as antagonists), a cell expressing c-met can be contacted with the compound to be screened for a particular activity associated with the function of c-met Sema domain, and the ability of the compound to inhibit the activity suggests that the compound could be an antagonist to the Sema domain (or sub-domain thereof), a property that could be further confirmed by determining its ability to bind or interact specifically with c-met Sema domain and not an unrelated molecule.

Other specific examples of potential antagonists include an oligonucleotide (which may be an aptamer) that binds to c-met Sema domain, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of c-met Sema domain that competitively inhibits the action of wild type c-met.

Potential antagonists include small molecules that bind to c-met Sema domain and/or the binding site of c-met Sema on its binding partner, thereby blocking the normal biological activity of the c-met Sema domain. Examples of small molecules include, but are not limited to, small peptides or peptide-like molecules, preferably soluble peptides, and synthetic non-peptidyl organic or inorganic compounds. These small molecules can be identified by any one of the screening assays discussed hereinabove and/or by any other screening techniques well known for those skilled in the art.

As described herein, an antagonist of the invention can be a peptide or polypeptide. Methods of obtaining such peptides or polypeptides are well known in the art, and include screening peptide or polypeptide libraries for binders to a suitable target antigen. In one embodiment, suitable target antigens would comprise at least a portion of c-met Sema domain. Libraries of peptides and polypeptides are well known in the art, and can also be prepared according to art methods. See, e.g., Clark et al., U.S. Pat. No. 6,121,416; Bass et al., U.S. Pat. No. 5,688,666. Libraries of peptides or polypeptides fused to a heterologous protein component, such as a phage coat protein, are well known in the art, e.g., as described in Clark et al., Bass et al., *supra.* In one embodiment, a peptide having ability to block c-met Sema domain function comprises the amino acid sequence LDAQT (SEQ ID NO: 1), LTEKRKKRS (SEQ ID NO:2), KPDSAEPM (SEQ ID NO:3) and/or NVRCLQHF (SEQ ID NO:4), or variants thereof. In one embodiment, a polypeptide having ability to block c-met Sema domain function comprises all or substantially all of the c-met Sema domain. Variants of a first peptide or polypeptide binder can be generated by screening mutants of the peptide or polypeptide to obtain the characteristics of interest (e.g., enhancing target binding affinity, enhanced pharmacokinetics, reduced toxicity, improved therapeutic index, etc.). Mutagenesis techniques are well known in the art. Furthermore, scanning mutagenesis techniques (such as those based on alanine scanning) can be especially helpful to assess structural and/or functional importance of individual amino acid residues within a peptide or polypeptide.

Determination of the ability of a candidate substance/molecule of the invention to modulate HGF/c-met signaling and/or biological activities associated with said signaling can be performed by testing the modulatory capability of the substance/molecule in *in vitro* or in *vivo* assays, which are well established in the art, e.g., as described in Okigaki et al., Biochemistry (1992), 31:9555-9561; Matsumoto et al., J. Biol. Chem. (1998), 273:22913-22920; Date et al., FEBS Let. (1997), 420:1-6; Lokker et al., EMBO J. (1992), 11:2503-2510; Hartman et al., Proc. Natl. Acad. Sci USA (1992), 89:11574-11578.

### Antibodies

The present invention further provides methods comprising use of antibodies that interfere with one or more processes necessary for c-met activation, for example c-met dimerization and/or binding of ligand to c-met. Exemplary antibodies include polyclonal, monoclonal, humanized, bispecific, and heteroconjugate antibodies.

### 1. Polyclonal Antibodies

Antibodies of the invention may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include a c-met Sema domain (or portion thereof) or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

Antibodies of the invention may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in *vitro.*

The immunizing agent will typically include the c-met Sema domain (or portion thereof) or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against c-met Sema domain. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). Hybridoma cells expressing an antibody of the invention would be a suitable source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

Antibodies can also be generated by screening phage display libraries for antibodies or antibody fragments that bind with suitable/desired affinity. Such techniques are well known in the art, e.g., as disclosed in U.S Pat. Nos. 5,750,373; 5,780,279; 5,821,047;6,040,136; 5,427,908; 5,580,717, and references therein.

### 3. Human and Humanized Antibodies

The antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

### 4. Bispecific Antibodies

Bispecific antibodies are monoclonal, preferably human or humanized, antibodies that have binding specificities for at least two different antigens. In the present case, one of the binding specificities is for c-met Sema domain, the other one is for any other antigen.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared can be prepared using chemical linkage. Brennan et aL, Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. *coli* and subjected to directed chemical coupling in *vitro* to form the bispecific antibody. The Bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies may bind to two different epitopes on a single c-met Sema domain or to an epitope on a c-met Sema domain and an epitope on another polypeptide.

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify an antibody of the invention with respect to effector function, so as to enhance, *e.g*., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced antitumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design. 3: 219-230 (1989).

### 7. Immunoconjugates

The invention also pertains to immunoconjugates comprising an antibody conjugated to a cytotoxic agent such as a chemotherapeutic agent, toxin (*e.g.,* an enzymatically active toxin of bacterial, fungal, plant, or animal origin, or fragments thereof), or a radioactive isotope (*i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I ¹³¹In, ⁹⁰Y, and ¹⁸⁶Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (*e.g*., avidin) that is conjugated to a cytotoxic agent (*e.g.,* a radionucleotide).

### 8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of an antibody of the present invention can be conjugated to the liposomes as described in Martin et al., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### 9. Pharmaceutical Compositions of Antibodies

Antibodies as well as other molecules identified by the screening assays disclosed hereinbefore, can be administered for the treatment of various disorders in the form of pharmaceutical compositions.

If whole antibodies are used as inhibitors, internalizing antibodies are preferred. However, lipofections or liposomes can also be used to deliver a substance/molecule of the invention into cells where that is desired. Where antibody fragments are used, it can be advantageous to use the smallest inhibitory fragment. For example, based upon the variable-region sequences of an antibody, peptide molecules can be designed that retain the ability to bind c-met Sema domain and/or interfere with interaction between c-met Sema domain and its binding partner. Such peptides can be synthesized chemically and/or produced by recombinant DNA technology. See, *e.g*., Marasco et al., Proc. Natl. Acad. Sci. USA. 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles, and nanocapsules) or in macroemulsions. Such techniques are disclosed in Remington's Pharmaceutical Sciences, *supra.*

The formulations to be used for in *vivo* administration must be sterile. This is readily accomplished by filtration through sterile filtration membranes.

Sustained-release preparations may be prepared. Suitable examples of sustained-release preparations include semipermeable matrices of solid hydrophobic polymers containing an antibody, which matrices are in the form of shaped articles, *e.g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is by itself or when combined with another composition is effective for treating, preventing and/or diagnosing the condition and may have a sterile access port (for example the container may be an intravenous solution bag or a vial having a stopper pierceable by a hypodermic injection needle). At least one active agent in the composition is an antagonist of the invention. The label or package insert indicates that the composition is used for treating the condition of choice, such as cancer. Moreover, the article of manufacture may comprise (a) a first container with a composition contained therein, wherein the composition comprises an antagonist of the invention; and (b) a second container with a composition contained therein, wherein the composition comprises a further cytotoxic agent The article of manufacture in this embodiment of the invention may further comprise a package insert indic ating that the first and second compositions can be used to treat a particular condition, e.g. cancer. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The following are examples of the methods and compositions of the invention. It is understood that various other embodiments may be practiced, given the general description provided above.

### EXAMPLES

### MATERIALS & METHODS

### Constructs and recombinant proteins

Extracellular sub-domain deletions of c-Met were constructed using conventional PCR methods. N-terminal primers containing the start of Sema, PSI, first IPT, or fourth IPT domains flanked by a KpnI site were paired with a C-terminal primer up to Met residue 959 flanked by a StuI site. c-Met was used as template and the PCR fragments for each clone were inserted into pCR-Blunt II-TOPO vector using the Zero Blunt TOPO PCR cloning kit (Invitrogen) according to manufacturer's instructions. The clones were confirmed by DNA sequencing. The constructs were then subcloned into pcDNA3.1 V5/His vector (Invitrogen) via KpnI and EcoRV to add a tag at the C-terminus. The signal peptide of Met was added via the HindIII and KpnI sites at the N-terminus of each clone. Each clone was digested with HindIII and EcoRV and subcloned into pRK5TKneo vector via HindIII and PmeL For EC-WT Flag and EC-WT V5/His clones, an N-terminal primer containing a HindIII site was paired with a C-terminal primer up to Met residue 595 flanked by a StuI site. EC-WT V5/His was subcloned via HindIII/StuI into the HindIII/EcoRV sites of pcDNA3.1 V5/His, and then subcloned into pRKSTKneo as described above. For EC-WT Flag, the PCR fragment was ligated to pCR-Blunt II-TOPO vector. Oligonucleotides containing a Flag tag sequence were inserted between StuI and KpnI. EC-WT Flag was then subcloned into pRK5TKneo via HindIII and EcoRV/ScaI. Recombinant HGF and anti-Met 5D5-Fab were provided by R. Schwall (Genentech). scHGF was produced in mammalian cells as described previously (Peek et al., 2002).

To obtain recombinant Sema and PSI domain, PCR was carried out on the region encoding residues 25 -567 of the MET receptor. An 8x His tag and NotI site were added to the C-terminus, and the N-terminus fused directly to the C-terminus of the insect cell secretion signal of pAcGP67A (BD Biosciences). The PCR product was digested with SpeI and NotI and subcloned into pAcGP67A. Purified plasmid DNA (pAcGP67A plus MET sema and PSI domain) was transfected into Sf9 insect cells according to the manufacturer's protocol (BD Biosciences). For expression, 1L of Hi5 insect cells growing in ESF 921 media (Protein Expression, LLC) at a density of 5 x 10⁵ cells/mL were infected with 10 mL of viral stock incubated at 27° C for 72 hours. Cells were then removed from the supernatant by centrifugation at 3000 g for 15 minutes. 1 mM of NiCl₂, 5 mM of CaCl₂, and 50 mM Tris pH 8.0 were added to the supernatant. Supernatant was filtered and applied to a 2 mL Ni-NTA column (Qiagen) by gravity flow, followed by 20 mL of wash buffer (50 mM Tris pH 8.0, 500 mM NaCl, 5 mM imidazole). Protein was eluted with a buffer containing 50 mM Tris 8.0, 300 mM NaCl, 250 mM imidazole. Attempts to make recombinant Sema domain alone did not yield protein as reported previously for Sema 3A (Antipenko et al., 2003).

### Immunoprecipitation and Western blot analysis

Cell lines were obtained from American Type Culture Collection (ATCC). 293 cells were maintained in DMEM supplemented with 10% FBS (Sigma), penicillin/streptomycin (GIBCO), and glutamine. A549, H441, and MDA-MB-435 cells were maintained in 50:50 DNEM/F12 (Cellgro) supplemented with 10% FBS, penicillin/streptomycin, and glutamine. Cells were transfected with 1-12 µg of the indicated constructs using Lipofectamine 2000 (Invitrogen) according to manufacturer's instructions. Twenty-four hrs later cells were harvested with 1% NP40 lysis buffer containing Complete protease inhibitor cocktail tablet and phosphatase inhibitor cocktail 2 (Roche and Sigma, respectively). For Met tyrosine phosphorylation and MAPK phosphorylation studies, cells were serum-starved for 2 hr with 0.5% BSA. Cells treated with 10 µg/ml anti-Met 5D5-Fab or rSema were incubated for an additional 1 hr prior to lysis. Cell debris was centrifuged and the supernatant was precleared with Protein A Sepharose (Sigma) or Protein G-Plus agarose beads (Santa Cruz) for 1 hr. 500 µg of lysates was then immunoprecipitated with 10 µg 5D5 antibody (Genentech) or 35 ul of Met (C-28) agarose conjugate (Santa Cruz) overnight at 4°C with rotation. For HGF binding studies, 500 µg of lysates were incubated with 5 µg scHGF or HGF followed by immunoprecipitation with 1 µg of V5 antibody. Protein A or G beads were added for an additional 2 hrs before immunoprecipitates were washed 3 times with 1% NP40 lysis buffer. After addition of 2X sample buffer with 10 mM DTT, the samples were run on 4-12% Tris-glycine gels (Invitrogen). Proteins were transferred to nitrocellulose membranes, blocked with 5% non-fat milk or BSA for 1 hour, and then probed with 1:5000 V5 (Invitrogen), 1:10000 C-terminal Met (C-12) (Santa Cruz), 1:1000 MAPK (Cell Signaling), 1:500 HGF-α (145) (Santa Cruz), 1:1000 P-MAPK (Cell Signaling), 1:1000 His (Cell Signaling), or 1:1000 phosphotyrosine 4G10 (Upstate) antibodies overnight at 4°C with rocking. Secondary antibodies against mouse or rabbit IgG were used at 1:5000 dilution (Amersham). Proteins were detected by enhanced chemiluminescence (ECL-Plus, Amersham).

### Covalent affinity crosslinking analysis

Crosslinking studies were performed as previously described with modifications (Blechman et al., 1995). A549, H441, MDA-MB-435, and 293 cells were serum starved for 2 hrs with 0.5% BSA. The media was removed and replaced with PBS. Cells were then treated with the crosslinking agent sulfo-EGS (Pierce) at increasing concentrations according to the manufacturer's instructions. Similarly, 293 cells were plated into 6-well plates and transfected with 0.1-4 µ3g of the indicated constructs using Lipofectamine 2000 (Invitrogen). Twenty-four hours later, the media was replaced with PBS and cells were crosslinked with sulfo-EGS. Cells were then lysed with 1% NP40 lysis buffer with protease and phosphatase inhibitors. 10 µg of lysates in reducing sample buffer were then analyzed immediately on 8% or 4-12% Tris-glycine gels, transferred to nitrocellulose membranes, and blocked with 5% milk for 1 hour. Membranes were probed with 1:1000 Met C-12 (Santa Cruz) or 1:5000 V5 antibody overnight at 4°C with rocking. After incubation of 1:5000 secondary antibodies, proteins were detected with ECL-Plus. EC-WT-Flag and EC-WT-V5/His constructs were co-transfected and crosslinked as before. 500 µg of crosslinked lysates were immunoprecipitated with either V5 or Flag antibody, analyzed on 8% Tris-glycine gels, transferred to nitrocellulose membranes, and immunoblotted with either 1:5000 V5 or 1:1000 Flag (Sigma) antibodies. Proteins were detected with ECL-Plus.

### Scrape assay

H441 cells were seeded at a density of 4.5 x 10⁴ cells/well in a 96-well plate with 10% FBS in 50:50 DMEM/F12. The next day, a single scrape was made in the confluent monolayer in each well as described previously (Lorenzato et al., 2002). The cells were then incubated with media containing 0.5% BSA and treated with mock, 10 µg/ml 5D5, 10 µg/ml Sema, or 100 ng/ml HGF. The scrape was monitored and photographed daily. Representative results are shown for Day 2.

### Migration assay

Transwell migration assays were carried out as previously described with modifications (Coltella et al., 2003). MDA-MB-435 cells were seeded at 1.2 x 10⁵ cells in media containing 0.5% BSA to the upper chamber of each transwell (Costar) that had been previously coated with 10 ug/ml collagen type IV (Sigma). Mock, 10 µg/ml anti-Met 5D5-Fab, or 10 µg/ml Sema was added to the upper chamber. As a positive control, 100 ng/ml HGF was also added to the lower chamber of mock-treated wells. The next day, the cells were fixed with 4% paraformaldehyde and stained with 0.5% crystal violet. Cells that had migrated to the lower chamber were solubilized with 10% acetic acid and the absorbance was measured at 560 nm in a microplate reader.

### RESULTS

### The Sema domain is necessary for Met receptor crosslinking

To determine the contribution made by the extracellular domain of Met to receptor dimerization and activation, sub-domain deletions of Met were made as shown in Figure 1. Each deletion mutant is flanked by the signal peptide (S.P.) at the N-terminus and a C-terminal transmembrane region carrying a V5/His tag. The Met deletion mutants were tested individually for the ability to crosslink using sulfo-EGS. Transfections of the V5/His tagged deletion mutants treated with increasing concentrations of sulfo-EGS were lysed and analyzed on 4-12% SDS-PAGE. In samples not treated with sulfo-EGS, EC-WT-V5/His appeared as processed monomer (EC-M, ∼ 90 kD) and unprocessed (*, ∼ 120 kD) proteins. EC-WT crosslinking was detected by a shift from the lower (EC-M, ∼90 kD) to an upper migratory dimer (EC-D, ∼180 kD) as shown in Figure 2A. Met mutants lacking the Sema domain did not display a similar shift. It is noted that as expected unprocessed EC-WT (*) Met did not display crosslinking as Sulfo-EGS is cell membrane impermeable. The intracellular single-chain precursor of Met is proteolytically cleaved when it is present on the cell surface (Giordano et al., 1989). Sulfo-EGS was selected as a crosslinking agent because of its limited permeability and the effects of crosslinking membrane-inserted processed Met receptor were examined.

To address if membrane-bound extracellular Met formed homodimers, cells were cotransfected with EC-WT-V5/His and EC-WT-Flag and crosslinked with sulfo-EGS. Cells were lysed, immunoprecipitated with V5 antibody, analyzed on 8% SDS-PAGE, and immunoblotted with either Flag or V5 antibodies. The immunoblots displayed an upper migratory form (EC-D, ∼180 kD) containing EC-WT-Flag and EC-WT-V5/His (Figure 2B), indicating that both forms of EC-WT were crosslinked and coimmunoprecipitated. A reverse immunoprecipitation using Flag antibody showed similar results (Figure 2B). Residual non-crosslinked EC-WT Flag (EC-M) was also detected in the V5 immunoprecipitates but shifted to the upper migratory form with the addition of increased sulfo-EGS (Figure 2B). Collectively, the crosslinking studies revealed that extracellular membrane-bound Met homodimers are formed when the sema domain is present.

### HGF and scHGF (R494E) bind to the Sema domain

HGF is secreted as an inactive single-chain precursor and is cleaved by extracellular proteases into an α and β chain (Naka et al., 1992). The cleaved disulfide linked α and β chains of HGF bind the Met receptor with high affinity (Bottaro et al., 1991; Naldini et al., 1991). The Met deletion mutants were tested for their ability to bind HGF. Cells were transfected with EC-WT, ΔS, ΔP, ΔIPT₃, TM or mock transfected (Figure 3A) and cell lysates were incubated with HGF. Immunoprecipitation with V5 antibody followed by 4-12% SDS-PAGE, and detection with α chain-HGF antibody indicated that HGF bound EC-WT alone (Figure 3B). The observation that HGF did not bind the remaining Met deletion mutants indicates that the Sema domain is necessary for HGF binding.

Single-chain HGF containing an R494E mutation [scHGF (R494E)] binds and inhibits activation of the Met receptor (Lokker et al., 1992). scHGF (R494E) was also examined for binding to the Met extracellular domain. Transfected Met deletion mutants were lysed, incubated with scHGF (R494E), and analyzed as above. The data indicated that scHGF (R494E) only binds EC-WT and not the Sema deleted mutants (Figure 3C). These observations were confirmed with coimmunoprecipitation experiments where both HGF and scHGF (R494E) bound to recombinant EC-Met and Sema domain proteins (data not shown). Thus, the data is consistent with the observation that the Sema domain of Met is the HGF interaction site (Gherardi et al., 2003). The data further indicate that the Sema domain of Met is the interaction site of scHGF (R494E) binding as well.

### 5D5 monoclonal antibody binds the Sema domain of Met

The Fab fragment of anti-Met 5D5 (anti-Met 5D5-Fab) has been shown to inhibit HGF/c-met signaling. See, for e.g., U.S. Pat. Nos. 5,686,292; 5,646,036; 6,207,152 & 6,214,344. The Met deletion constructs were tested in co-immunoprecipitation experiments with anti-Met 5D5. Cells were transfected with the Met deletion constructs or mock transfected as before. These cell lysates were immunoprecipitated with anti-Met 5D5 and immunoblotted with His antibody. As shown in Figure 3E, only the Sema-containing EC-WT was bound to anti-Met 5D5. All transfected proteins were detected in the lysates (Figure 3D), and endogenous Met was immunoprecipitated by anti-Met 5D5 (Figure 3F). Thus, the data indicate that anti-Met 5D5 binds the Sema domain of Met. Since anti-Met 5D5-Fab acts as an antagonist, it was used as a control in the following functional studies.

### Crosslinking of endogenous Met in tumor cell lines

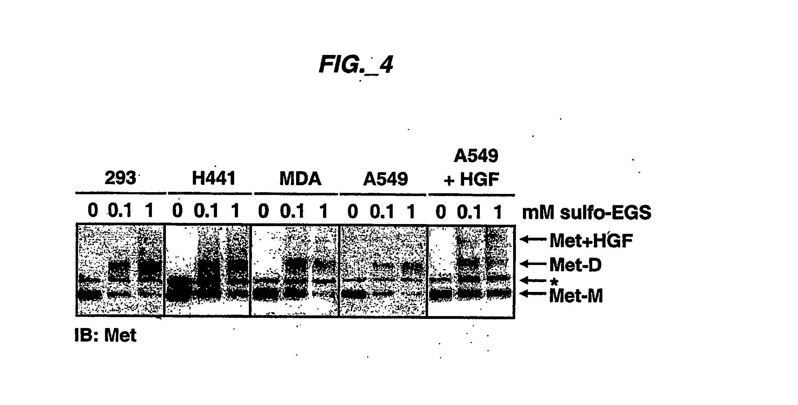

Since the data indicated that the Sema domain is associated with two functions, namely HGF binding and receptor dimerization, the role of the Met Sema domain in dimerization excluding involvement of HGF binding was examined. Unless indicated otherwise, all subsequent studies were carried out in the absence of HGF. The levels of HGF expressed in 293 (embryonic kidney), H441 (lung adenocarcinoma), A549 (lung carcinoma), and MDA-MB-435 (breast carcinoma) cell lines were determined by RT-PCR and Western blot analysis. MDA-MB-435 and H441 cells did not have detectable HGF RNA or protein, while A549 and 293 cells expressed HGF RNA and had barely detectable HGF protein in lysates and conditioned media (data not shown). To determine if Met expressed in various tumor cell lines could crosslink in the absence of HGF, cells were maintained in serum-free media containing 0.5% BSA for the duration of the experiment. These cell lines were crosslinked with sulfo-EGS as before and lysates were analyzed by 4-12% SDS-PAGE followed by immunoblot with Met antibody. In the absence of crosslinking reagent, Met appeared as processed (Met-M) and unprocessed (*) proteins as previously seen in crosslinking studies with EC-WT (Figure 2A). In each cell line, even at 0.1 mM sulfo-EGS a shift from Met-M to an upper migratory form (Met-D) was observed (Figure 4). Increased concentrations of crosslinking agent enhanced the shift of Met-M to the Met D form. Consistent with our previous observation, intracellular unprocessed Met did not crosslink upon sulfo-EGS treatment. In comparison, A549 cells were incubated with 100 ng/ml HGF and crosslinked as before. Immunoblotting for Met revealed a higher migratory form in the presence of HGF when compared to Met-D seen in the absence of HGF (Figure 4). The data indicated that crosslinked Met dimers were formed in the absence of HGF and were shifted further due to HGF binding.

### Inhibition of Met signaling in tumor cells with rSema and anti-Met 5D5-Fab

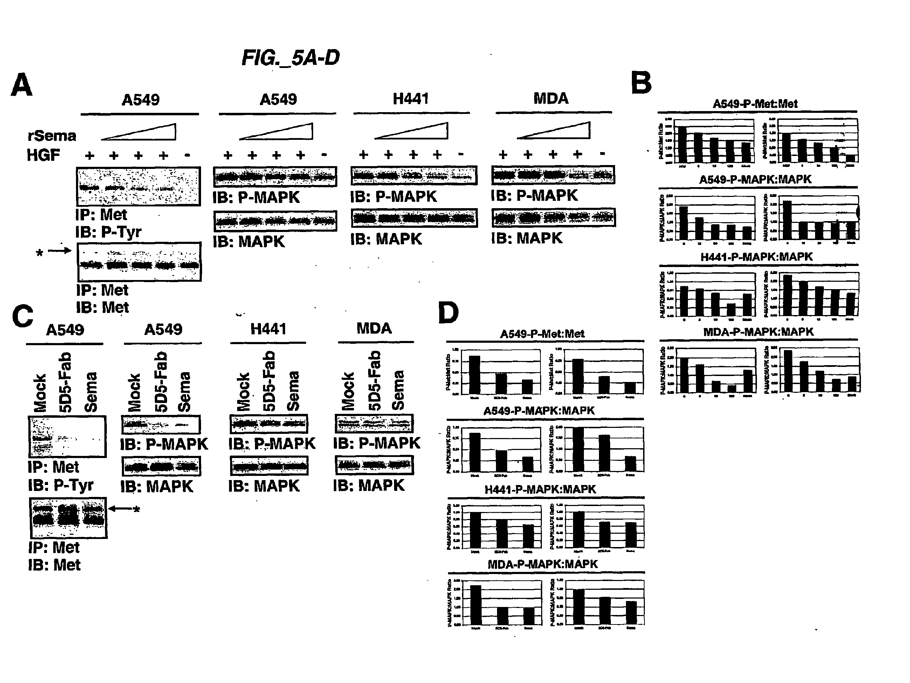

Since the data indicated that the Sema domain is necessary for Met dimerization, recombinant Sema (rSema) protein, which included the Sema and PSI domains of the Met receptor (see Materials and Methods section) was generated, and the functional consequences on Met signaling examined. Human tumor cells were treated with increasing concentrations of rSema and analyzed for Met tyrosine phosphorylation and downstream activation of mitogen activated protein kinase (MAPK) upon HGF stimulation. A549 cells were serum starved and incubated with increasing concentrations of rSema in the presence of HGF. Cell lysates were harvested, immunoprecipitated with a C-terminal Met antibody, and immunoblotted with phospho-tyrosine antibody. A decrease in Met phosphorylation was observed with increasing amounts of rSema in the presence of HGF compared to HGF stimulation alone (Figure 5A and 5B). Similarly, phosphorylation of MAPK decreased upon treatment with increasing concentrations of rSema in the presence of ligand. H441 and MDA-MB-435 cell lines were also treated with rSema and HGF in a similar manner and a dose dependent decrease in phospho-MAPK was observed (Figure 5A and 5B).

In addition, inhibition of Met activation by rSema in the absence of HGF was also examined. For comparison, anti-Met 5D5-Fab that acts as a Met receptor antagonist was used in the functional studies. A549 cells were serum-starved and treated with anti-Met 5D5-Fab or rSema. Treatment with rSema or anti-Met 5D5-Fab caused an inhibition of ligand independent Met phosphorylation and phospho-MAPK levels compared to mock treated control (Figure 5C and 5D). Similarly, H441 and MDA-MB-435 cell lines were also treated with anti-Met 5D5-Fab and rSema in the absence of HGF (Figure 5C and 5D); the decrease in MAPK phosphorylation followed a trend similar to the previous observations with A549 cells. These results suggested that rSema not only inhibits ligand dependent and independent Met phosphorylation but also affects downstream MAPK signaling.

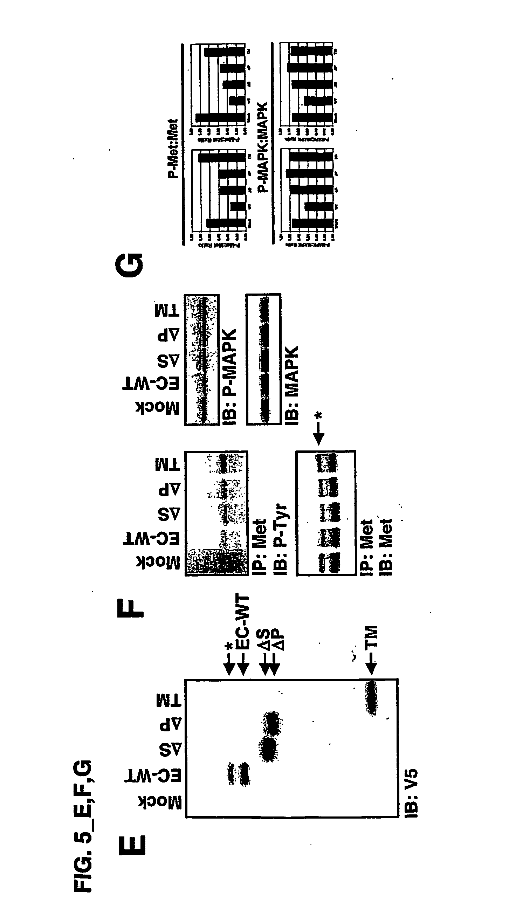

Met deletion mutants were also examined for their ability to attenuate endogenous Met signal transduction. Cells were transfected with the Met deletion mutants (Figure 5E); lysates were immunoprecipitated with a C-terminal Met antibody and immunoblotted with phosphotyrosine antibody. EC-WT transfectants showed attenuation of Met tyrosine phosphorylation and phospho-MAPK compared to mock treated controls (Figure 5F and 5G). Sema deleted transfectants, while showing some attenuation of phospho-Met, did not show a decrease in downstream MAPK phosphorylation (Figure 5F and 5G). Together, these observations confirmed that the Sema domain is necessary for Met activation, corroborating the previous results with rSema inhibition of Met signaling (Figure 5A-D).

### Cell migration is inhibited by rSema and anti-Met 5D5-Fab

H441 cells were used in a scrape assay as a measure of Met mediated cell motility (Lorenzato et al., 2002). H441 cells grown to high density were scraped in each well on day 0 and maintained in serum-free media containing 0.5% BSA for the duration of the experiment (Figure 6). Cells were either treated with 10 µg/ml rSema or 5D5 or 100 ng/ml HGF as a positive control or mock treated. On Day 2, the gap in mock treated wells closed completely while a gap remained in the rSema treated cells (Figure 6). The gap in anti-Met 5D5 Fab treated cells also remained visible, to a lesser extent. HGF treated cells had closed the gap by Day 1 and remained as such on Day 2. These observations were consistent in four separate experiments. The data suggested that cell motility mediated by endogenous Met activation is inhibited by both rSema and anti-Met 5D5-Fab in the absence of HGF.

To examine the effect of rSema on cell motility in the presence of ligand, cells were treated with 0, 0.1, 0.5, or 5 µg/ml rSema plus 20 ng/ml HGF. Cells treated with HGF alone closed the gap completely by Day 1. In comparison, cells treated with rSema in the presence of HGF retained the gap in a dose dependent manner (data not shown). The data suggested that rSema inhibits Met-mediated cell motility in the presence of HGF.

A transwell assay with MDA-MB-435 cells was used to measure Met driven cell migration (Coltella et al., 2003). In the absence of HGF, mock treated cells migrated as indicated in Figure 7. This migration was consistently decreased by the addition of rSema, and anti-Met 5D5-Fab to a lesser extent. Addition of HGF to these cells resulted in ∼3-fold increase in migration. The data correlated with the scrape assay results in the H441 cell line. Activation of Akt was also examined in H441 and MDA-MB-435 cells used in the cell motility and migration assays. A dose dependent decrease in Akt phosphorylation with increasing concentrations of rSema in the presence of HGF was observed (data not shown). The data correlated with observations that rSema can inhibit cell motility in the presence of ligand. Taken together, these observations supports the conclusion that a Sema domain antagonist, such as a recombinant polypeptide comprising a Sema sequence, is capable of not only inhibiting Met activation, but also blocking downstream effects of cell motility and migration in both ligand dependent and independent contexts.

### Discussion

The Sema domain resides in the extracellular region of semaphorins and their receptors, plexins, and serves as a receptor/ligand recognition site (Tamagnone et al., 1999). Recent publications of the crystal structures of Sema 3A and Sema 4D suggest that these Sema domains form seven-bladed β-propeller structures that are important for homodimerization (Antipenko et al., 2003; Love et al., 2003). In vitro and in vivo experimental evidence described herein strongly support a role for the Sema domain in Met receptor dimerization.

The data show that crosslinking of Met occurs only in the presence of the Sema domain, suggesting a necessary role for the Sema domain in receptor dimerization. In addition, crosslinking of Met was observed in the absence of HGF, indicating that Met receptor in these tumor cell lines may dimerize without ligand stimulation.

The crystal structure of Sema3A suggests that four interaction "loops" in the Sema domain are important to establish the interface between Sema3A dimers (Antipenko et al., 2003). Alignment of the Sema3A sequence with Met reveals that one "loop" resides close to the proteolytic cleavage site between R307 and S308 in the Met Sema domain, which may suggest its importance in Met dimerization.

The cell-based study described herein reveals Sema domain mediated homophilic interactions of the Met receptor in the absence of HGF. Notably, previous reports suggest that Met also interacts with CD44v6, α6β4 integrin, and plexin B1 (Giordano et al., 2002; Orian-Rousseau et al., 2002; Trusolino et al., 2001). The results described herein provides support for the notion that Met association with plexin B1 could occur through these highly conserved Sema domains. In addition, overexpression of both Met and semaphorins has been described in cancers and invasive metastases, leading to further speculation that any potential interactions could be mediated through the Sema domains. However, the role of these molecules and the mechanisms that mediate heteromeric interactions in these pathological contexts would benefit from further investigation. CD44v6 and α6β4 are not known to contain Sema domains. Therefore, interactions with other protein motifs mediated through the Sema and/or the PSI or IPT domains may be important in initiating specific biological responses (Bertotti and Comoglio, 2003) and cannot be excluded as a possibility.

In RTKs such as fibroblast growth factor receptor 2 (FGFR2) and RET, disulfide bonding between cysteines in the extracellular domain of each receptor have been implicated in receptor dimerization (Robertson et al., 2000). In the Met receptor, the data described herein show that neither the cysteine-rich PSI domain nor the four IPT domains exhibit crosslinking in the absence of the Sema domain, suggesting that these regions may not be necessary for Met dimerization. Furthermore, Met interaction with plexin B1 was unaltered despite deletion of the PSI domain of Met (Giordano et al., 2002). Although the rSema used in the functional studies described herein contained a Sema and PSI domain (refer to Material and Methods), the crosslinking studies described herein and the reported PSI domain deletion in plexin B1 (Giordano et al., 2002) strongly indicate that the Sema domain plays a dominant role in these interactions in the absence of the PSI domain. It is noted that attempts to generate internal PSI or IPT deletions resulted in expression of non-processed Met that could not be used in the present study, and therefore, it remains to be definitively shown whether or not there is a role for the PSI and IPT domains in Met dimer formation or some other mechanism such as auto-inhibition of dimerization as reported for EGFR (Ferguson et al., 2003). Resolution of the crystal structures of extracellular Met would shed more light on these complex interactions.

Crystallographic studies of ligand-receptor complexes of RTKs have revealed several structural insights. Both Flt-1 and TrkA utilize dimerization of ligands for receptor dimerization and activation (Wiesmann et al., 1997; Wiesmann et al., 1999). In FGFR, heparin bound FGF ligands drive receptor dimerization (Plotnikov et al., 1999). In contrast, the EGF:EGFR (epidermal growth factor receptor) complex homodimerizes through the receptor, independent of ligand-ligand interaction (Garrett et al., 2002; Ogiso et al., 2002). The interaction of Met and HGF is less clear, although a recent report suggests that Met, HGF, and heparin exist as a 1:1:1 complex (Gherardi et al., 2003). Our cell-based studies differ from these reported in vitro observations since cellular components such as the extracellular matrix (ECM) are present in our study and may have considerable influence on receptor dimerization *in vivo.* Since the ECM contains laminins, and other components responsible for cellular interactions (Giancotti and Ruoslahti, 1999), it is likely that all proteins which facilitate Met dimerization may not yet be identified.

As demonstrated herein, the Sema domain is sufficient for interaction with HGF, consistent with previous observations (Gherardi et al., 2003), and rSema is capable of inhibiting ligand driven Met activation. Thus, it is likely that rSema would effectively inhibit HGF-dependent Met activation by binding HGF. Furthermore, it suggests that HGF dependent Met overexpressing tumors could also be targeted by rSema in a similar manner to Ron Sema domain mediated inhibition of ligand-dependent receptor activation (Angeloni et al., 2003). The data described herein indicate that the Sema-HGF interaction site differs from the Met dimerization interface since the crosslinking studies show that ligand-independent Met dimers shift further when bound to HGF (Figure 4). These data imply that the dimerization interface and the HGF interaction site are non-overlapping.

In addition, it is demonstrated herein that the Sema domain is necessary for binding of a known c-met antagonist antibody, anti-Met 5D5-Fab. Since anti-Met 5D5-Fab also inhibits ligand-independent receptor activation as observed in the findings described herein, it is possible that in addition to HGF competition anti-Met 5D5-Fab may block receptor dimerization by steric hindrance. Likewise, rSema also inhibits ligand independent and dependent Met activity indicating a role for rSema as an effective Met inhibitor. Similar inhibitory activity has been described for the c-kit receptor (Lev et al., 1992). The role of the Met Sema domain in dimerization in a ligand independent context addressed in this study differs from the role of the Ron sema domain. While the Ron Sema domain competes for binding with MSP to inhibit Ron activity it does not inhibit Ron receptor dimerization (Angeloni et al., 2003). The functional studies described herein were carried out in the presence or absence of HGF, demonstrating that the Met Sema domain inhibits both ligand dependent and independent receptor activation. These data suggest that although the structural motifs and binding interactions of Met and Ron may be similar, the *in vivo* contexts may specify differing homophilic interactions.

It is demonstrated herein that the Met Sema domain is a potential inhibitor of not only ligand dependent but also ligand-independent Met receptor activation. The data described herein indicate that the Sema domain of Met blocks receptor activation in tumor cells, whereby MAPK phosphorylation, cell motility and migration are inhibited. These observations point to the possibility of treating tumors associated with dysregulation of the HGF/c-met signaling axis by targeting the function of the Sema domain of Met, including but not limited to utilizing the Sema domain itself as a biotherapeutic.

### PARTIAL REFERENCE LIST

Angeloni, D., Danilkovitch-Miagkova, A., Miagkov, A., Leonard, E. J., and Lerman, M. I. (2003). The soluble sema domain of the Ron receptor inhibits MSP-induced receptor activation. J Biol Chem.
Antipenko, A., Himanen, J. P., van Leyen, K., Nardi-Dei, V., Lesniak, J., Barton, W. A., Rajashankar, K. R., Lu, M., Hoemme, C., Puschel, A. W., and Nikolov, D.B. (2003). Structure of the semaphorin-3A receptor binding module. Neuron 39, 589-598.
Bardelli, A., Longati, P., Gramaglia, D., Stella, M. C., and Comoglio, P. M. (1997). Gab1 coupling to the HGF/Met receptor multifunctional docking site requires binding of Grb2 and correlates with the transforming potential. Oncogene 15, 3103-3111.
Bertotti, A., and Comoglio, P. M. (2003). Tyrosine kinase signal specificity: lessons from the HGF receptor. Trends Biochem Sci 28, 527-533.
Bladt, F., Riethmacher, D., Isenmann, S., Aguzzi, A., and Birchmeier, C. (1995). Essential role for the c-met receptor in the migration of myogenic precursor cells into the limb bud. Nature 376, 768-771.
Blechman, J. M., Lev, S., Barg, J., Eisenstein, M., Vaks, B., Vogel, Z., Givol, D., and Yarden, Y. (1995). The fourth immunoglobulin domain of the stem cell factor receptor couples ligand binding to signal transduction. Cell 80, 103-113.
Boix, L., Rosa, J. L., Ventura, F., Castells, A., Bruix, J., Rodes, J., and Bartrons, R. (1994). c-met mRNA overexpression in human hepatocellular carcinoma. Hepatology 19, 88-91.
Bottaro, D. P., Rubin, J. S., Faletto, D. L., Chan, A. M., Kmiecik, T. E., Vande Woude, G. F., and Aaronson, S. A. (1991). Identification of the hepatocyte growth factor receptor as the c-met protooncogene product. Science 251, 802-804.
Bussolino, F., Di Renzo, M. F., Ziche, M., Bocchietto, E., Olivero, M., Naldini, L., Gaudino, G., Tamagnone, L., Coffer, A., and Comoglio, P. M. (1992). Hepatocyte growth factor is a potent angiogenic factor which stimulates endothelial cell motility and growth. J Cell Biol 119, 629-641.
Coltella, N., Manara, M. C., Cerisano, V., Trusolino, L., Di Renzo, M. F., Scotlandi, K., and Ferracini, R. (2003). Role of the MET/HGF receptor in proliferation and invasive behavior of osteosarcoma. Faseb J 17, 1162-1164.
Cooper, C. S., Park, M., Blair, D. G., Tainsky, M. A., Huebner, K., Croce, C. M., and Vande Woude, G. F. (1984). Molecular cloning of a new transforming gene from a chemically transformed human cell line. Nature 311, 29-33.
Di Renzo, M. F., Olivero, M., Giacomini, A., Porte, H., Chastre, E., Mirossay, L., Nordlinger, B., Bretti, S., Bottardi, S., Giordano, S., and et al. (1995). Overexpression and amplification of the met/HGF receptor gene during the progression of colorectal cancer. Clin Cancer Res 1, 147-154.
Ferguson, K. M., Berger, M. B., Mendrola, J. M., Cho, H. S., Leahy, D. J., and Lemmon, M. A. (2003). EGF activates its receptor by removing interactions that autoinhibit ectodomain dimerization. Mol Cell 11, 507-517.
Furge, K. A., Zhang, Y. W., and Vande Woude, G. F. (2000). Met receptor tyrosine kinase: enhanced signaling through adapter proteins. Oncogene 19, 5582-5589.
Garrett, T. P., McKern, N. M., Lou, M., Elleman, T. C., Adams, T. E., Lovrecz, G. 0., Zhu, H. J., Walker, F., Frenkel, M. J., Hoyne, P. A., et al. (2002). Crystal structure of a truncated epidermal growth factor receptor extracellular domain bound to transforming growth factor alpha. Cell 110, 763-773.
Gherardi, E., Youles, M. E., Miguel, R. N., Blundell, T. L., lamele, L., Gough, J., Bandyopadhyay, A., Hartmann, G., and Butler, P. J. (2003). Functional map and domain structure of MET, the product of the c-met protooncogene and receptor for hepatocyte growth factor/scatter factor. Proc Natl Acad Sci USA.
Giancotti, F. G., and Ruoslahti, E. (1999). Integrin signaling. Science 285, 1028-1032.
Giordano, S., Corso, S., Conrotto, P., Artigiani, S., Gilestro, G., Barberis, D., Tamagnone, L., and Comoglio, P. M. (2002). The semaphorin 4D receptor controls invasive growth by coupling with Met. Nat Cell Biol 4, 720-724.
Giordano, S., Di Renzo, M. F., Narsimhan, R. P., Cooper, C. S., Rosa, C., and Comoglio, P. M. (1989). Biosynthesis of the protein encoded by the c-met proto-oncogene. Oncogene 4, 1383-1388.
Giordano, S., Maffe, A., Williams, T. A., Artigiani, S., Gual, P., Bardelli, A., Basilico, C., Michieli, P., and Comoglio, P. M. (2000). Different point mutations in the met oncogene elicit distinct biological properties. Faseb J 14, 399-406.
Hamanoue, M., Takemoto, N., Matsumoto, K., Nakamura, T., Nakajima, K., and Kohsaka, S. (1996). Neurotrophic effect of hepatocyte growth factor on central nervous system neurons in vitro. J Neurosci Res 43, 554-564.
Hartmann, G., Weidner, K. M., Schwarz, H., and Birchmeier, W. (1994). The motility signal of scatter factor/hepatocyte growth factor mediated through the receptor tyrosine kinase met requires intracellular action of Ras. J Biol Chem 269, 21936-21939.
Jeffers, M., Rong, S., and Vande Woude, G. F. (1996). Enhanced tumorigenicity and invasion-metastasis by hepatocyte growth factor/scatter factor-met signalling in human cells concomitant with induction of the urokinase proteolysis network. Mol Cell Biol 16, 1115-1125.
Jeffers, M., Schmidt, L., Nakaigawa, N., Webb, C. P., Weirich, G., Kishida, T., Zbar, B., and Vande Woude, G. F. (1997). Activating mutations for the met tyrosine kinase receptor in human cancer. Proc Natl Acad Sci U S A 94, 11445-11450.
Jin, L., Fuchs, A., Schnitt, S. J., Yao, Y., Joseph, A., Lamszus, K., Park, M., Goldberg, L D., and Rosen, E. M. (1997). Expression of scatter factor and c-met receptor in benign and malignant breast tissue. Cancer 79, 749-760.
Kuniyasu, H., Yasui, W., Yokozaki, H., Kitadai, Y., and Tahara, E. (1993). Aberrant expression of c-met mRNA in human gastric carcinomas. Int J Cancer 55, 72-75.
Lev, S., Yarden, Y., and Givol, D. (1992). A recombinant ectodomain of the receptor for the stem cell factor (SCF) retains ligand-induced receptor dimerization and antagonizes SCF-stimulated cellular responses. J Biol Chem 267, 10866-10873.
Liu, C., Park, M., and Tsao, M. S. (1992). Overexpression of c-met proto-oncogene but not epidermal growth factor receptor or c-erbB-2 in primary human colorectal carcinomas. Oncogene 7, 181-185.
Lokker, N. A., Mark, M. R., Luis, E. A., Bennett, G. L., Robbins, K. A., Baker, J. B., and Godowski, P. J. (1992). Structure-function analysis of hepatocyte growth factor: identification of variants that lack mitogenic activity yet retain high affinity receptor binding. Embo J 11, 2503-2510.
Lorenzato, A., Olivero, M., Patane, S., Rosso, E., Oliaro, A., Comoglio, P. M., and Di Renzo, M. F. (2002). Novel somatic mutations of the MET oncogene in human carcinoma metastases activating cell motility and invasion. Cancer Res 62, 7025-7030.
Love, C. A., Harlos, K., Mavaddat, N., Davis, S. J., Stuart, D. L, Jones, E. Y., and Esnouf, R. M. (2003). The ligand-binding face of the semaphorins revealed by the high-resolution crystal structure of SEMA4D. Nat Struct Biol 10,843-848.
Maina, F., Casagranda, F., Audero, E., Simeone, A., Comoglio, P. M., Klein, R., and Ponzetto, C. (1996). Uncoupling of Grb2 from the Met receptor in vivo reveals complex roles in muscle development. Cell 87, 531-542.
Matsumoto, K., and Nakamura, T. (1993). Roles of HGF as a pleiotropic factor in organ regeneration. Exs 65, 225-249.
Maulik, G., Shrikhande, A., Kijima, T., Ma, P. C., Morrison, P. T., and Salgia, R. (2002). Role of the hepatocyte growth factor receptor, c-Met, in oncogenesis and potential for therapeutic inhibition. Cytokine Growth Factor Rev 13, 41-59.
Meiners, S., Brinkmann, V., Naundorf, H., and Birchmeier, W. (1998). Role of morphogenetic factors in metastasis of mammary carcinoma cells. Oncogene 16, 9-20.
Morello, S., Olivero, M., Aimetti, M., Bernardi, M., Berrone, S., Di Renzo, M. F., and Giordano, S. (2001). MET receptor is overexpressed but not mutated in oral squamous cell carcinomas. J Cell Physiol 189, 285-290.
Naka, D., Ishii, T., Yoshiyama, Y., Miyazawa, K., Hara, H., Hishida, T., and Kidamura, N. (1992). Activation of hepatocyte growth factor by proteolytic conversion of a single chain form to a heterodimer. J Biol Chem 267, 20114-20119.
Naldini, L., Weidner, K. M., Vigna, E., Gaudino, G., Bardelli, A., Ponzetto, C., Narsimhan, R. P., Hartmann, G., Zarnegar, R., Michalopoulos, G. K., and et al. (1991). Scatter factor and hepatocyte growth factor are indistinguishable ligands for the MET receptor. Embo J 10, 2867-2878.
Natali, P. G., Prat, M., Nicotra, M. R., Bigotti, A., Olivero, M., Comoglio, P. M., and Di Renzo, M. F. (1996). Overexpression of the met/HGF receptor in renal cell carcinomas. Int J Cancer 69, 212-217.
Nguyen, L., Holgado-Madruga, M., Maroun, C., Fixman, E. D., Kamikura, D., Fournier, T., Charest, A., Tremblay, M. L., Wong, A. J., and Park, M. (1997). Association of the multisubstrate docking protein Gab1 with the hepatocyte growth factor receptor requires a functional Grb2 binding site involving tyrosine 1356. J Biol Chem 272, 20811-20819.
Nusrat, A., Parkos, C. A., Bacarra, A. E., Godowski, P. J., Delp-Archer, C., Rosen, E. M., and Madara, J. L. (1994). Hepatocyte growth factor/scatter factor effects on epithelia. Regulation of intercellular junctions in transformed and nontransformed cell lines, basolateral polarization of c-met receptor in transformed and natural intestinal epithelia, and induction of rapid wound repair in a transformed model epithelium. J Clin Invest 93, 2056-2065.
Ogiso, H., Ishitani, R., Nureki, O., Fukai, S., Yamanaka, M., Kim, J. H., Saito, K, Sakamoto, A., Inoue, M., Shirouzu, M., and Yokoyama, S. (2002). Crystal structure of the complex of human epidermal growth factor and receptor extracellular domains. Cell 110, 775-787.
Olivero, M., Rizzo, M., Madeddu, R., Casadio, C., Pennacchietti, S., Nicotra, M. R., Prat, M., Maggi, G., Arena, N., Natali, P. G., et al. (1996). Overexpression and activation of hepatocyte growth factor/scatter factor in human non-small-cell lung carcinomas. Br J Cancer 74, 1862-1868.
Olivero, M., Valente, G., Bardelli, A., Longati, P., Ferrero, N., Cracco, C., Terrone, C., Rocca-Rossetti, S., Comoglio, P. M., and Di Renzo, M. F. (1999). Novel mutation in the ATP-binding site of the MET oncogene tyrosine kinase in a HPRCC family. Int J Cancer 82, 640-643.
Orian-Rousseau, V., Chen, L., Sleeman, J. P., Herrlich, P., and Ponta, H. (2002). CD44 is required for two consecutive steps in HGF/c-Met signaling. Genes Dev 16, 3074-3086.
Park, M., Dean, M., Cooper, C. S., Schmidt, M., O'Brien, S. J., Blair, D. G., and Vande Woude, G. F. (1986). Mechanism of met oncogene activation. Cell 45, 895-904.
Peek, M., Moran, P., Mendoza, N., Wickramasinghe, D., and Kirchhofer, D. (2002). Unusual proteolytic activation of pro-hepatocyte growth factor by plasma kallikrein and coagulation factor XIa. J Biol Chem 277,47804-47809.
Pelicci, G., Giordano, S., Zhen, Z., Salcini, A. E., Lanfrancone, L., Bardelli, A., Panayotou, G., Waterfield, M. D., Ponzetto, C., Pelicci, P. G., and et al. (1995). The motogenic and mitogenic responses to HGF are amplified by the Shc adaptor protein. Oncogene 10, 1631-1638.
Plotnikov, A. N., Schlessinger, J., Hubbard, S. R., and Mohammadi, M. (1999). Structural basis for FGF receptor dimerization and activation. Cell 98, 641-650.
Ponzetto, C., Bardelli, A., Zhen, Z., Maina, F., dalla Zonca, P., Giordano, S., Graziani, A., Panayotou, G., and Comoglio, P. M. (1994). A multifunctional docking site mediates signaling and transformation by the hepatocyte growth factor/scatter factor receptor family. Cell 77, 261-271.
Ponzetto, C., Zhen, Z., Audero, E., Maina, F., Bardelli, A., Basile, M. L., Giordano, S., Narsimhan, R., and Comoglio, P. (1996). Specific uncoupling of GRB2 from the Met receptor. Differential effects on transformation and motility. J Biol Chem 271, 14119-14123.
Robertson, S. C., Tynan, J. A., and Donoghue, D. J. (2000). RTK mutations and human syndromeswhen good receptors turn bad. Trends Genet 16, 265-271.
Royal, L, and Park, M. (1995). Hepatocyte growth factor-induced scatter of Madin-Darby canine kidney cells requires phosphatidylinositol 3-kinase. J Biol Chem 270, 27780-27787.
Schmidt, C., Bladt, F., Goedecke, S., Brinkmann, V., Zschiesche, W., Sharpe, M., Gherardi, E., and Birchmeier, C. (1995). Scatter factor/hepatocyte growth factor is essential for liver development. Nature 373, 699-702.
Schmidt, L., Duh, F. M., Chen, F., Kishida, T., Glenn, G., Choyke, P., Scherer, S. W., Zhuang, Z., Lubensky, L, Dean, M., et al. (1997). Germline and somatic mutations in the tyrosine kinase domain of the MET proto-oncogene in papillary renal carcinomas. Nat Genet 16, 68-73.
Schmidt, L., Junker, K., Nakaigawa, N., Kinjerski, T., Weirich, G., Miller, M., Lubensky, I., Neumann, H. P., Brauch, H., Decker, J., et al. (1999). Novel mutations of the MET proto-oncogene in papillary renal carcinomas. Oncogene 18, 2343-2350.
Suzuki, K., Hayashi, N., Yamada, Y., Yoshihara, H., Miyamoto, Y., Ito, Y., Ito, T., Katayama, K., Sasaki, Y., Ito, A., and et al. (1994). Expression of the c-met protooncogene in human hepatocellular carcinoma. Hepatology 20, 1231-1236.
Tamagnone, L., Artigiani, S., Chen, H., He, Z., Ming, G. I., Song, H., Chedotal, A., Winberg, M. L., Goodman, C. S., Poo, M., et al. (1999). Plexins are a large family of receptors for transmembrane, secreted, and GPI-anchored semaphorins in vertebrates. Cell 99, 71-80.
Tempest, P. R., Stratton, M. R., and Cooper, C. S. (1988). Structure of the met protein and variation of met protein kinase activity among human tumour cell lines. Br J Cancer 58, 3-7.
Trusolino, L., Bertotti, A., and Comoglio, P. M. (2001). A signaling adapter function for alpha6beta4 integrin in the control of HGF-dependent invasive growth. Cell 107, 643-654.
Uehara, Y., Minowa, O., Mori, C., Shiota, K., Kuno, J., Noda, T., and Kitamura, N. (1995). Placental defect and embryonic lethality in mice lacking hepatocyte growth factor/scatter factor. Nature 373, 702-705.
Van Vactor, D. V., and Lorenz, L. J. (1999). Neural development: The semantics of axon guidance. Curr Biol 9, R201-204.
Weidner, K. M., Di Cesare, S., Sachs, M., Brinkmann, V., Behrens, J., and Birchmeier, W. (1996). Interaction between Gab1 and the c-Met receptor tyrosine kinase is responsible for epithelial morphogenesis. Nature 384, 173-176.
Wiesmann, C., Fuh, G., Christinger, H. W., Eigenbrot, C., Wells, J. A., and de Vos, A. M. (1997). Crystal structure at 1.7 A resolution of VEGF in complex with domain 2 of the Flt-1 receptor. Cell 91, 695-704.
Wiesmann, C., Ultsch, M. H., Bass, S. H., and de Vos, A. M. (1999). Crystal structure of nerve growth factor in complex with the ligand-binding domain of the TrkA receptor. Nature 401, 184-188.

The following numbered paragraphs (paras.) contain further statements of various aspects of the present invention:-
1. A c-met antagonist that disrupts c-met dimerization.
2. The antagonist of para. 1 which disrupts dimerization function of c-met Sema domain.
3. The antagonist of para. 1 which binds to c-met such that c-met dimerization is disrupted.
4. The antagonist of para. 1 which binds to c-met such that ability of c-met Sema domain to effect c-met dirnerization is disrupted.
5. A c-met antagonist that specifically binds a sequence in the c-met Sema domain.
6. The antagonist of para. 1 or 5 which upon binding to a c-met molecule inhibits dimerization of said molecule.
7. The antagonist of para. 6 wherein said dimerization comprises homodimerization.
8. The antagonist of any of paras. 1-7 which does not bind an HGF binding site on c-met.
9. The antagonist of any of paras. 1-8 which does not substantially compete with hepatocyte growth factor (HGF) for binding to c-met.
10. The antagonist of any of paras. 1-9 which does not substantially inhibit binding of hepatocyte growth factor to c-met.
11. The antagonist of any of paras. 1-8 which competes with HGF for binding to c-met.
12. The antagonist of any of paras. 1-11 which binds to an epitope on c-met distinct from an epitope to which the monoclonal antibody produced by the hybridoma cell line deposited under American Type Culture Collection Accession Number ATCC HB 11894 (hybridoma 1A3.3.13) or HB-11895 (hybridoma 5D5.11.6) binds.
13. The antagonist of any of paras. 1-11 which is not the monoclonal antibody produced by the hybridoma cell line deposited under American Type Culture Collection Accession Number ATCC HB-11894 (hybridoma 1A3.3.13) or HB-11895 (hybridoma 5D5.11.6) binds.
14. The antagonist of any of paras. 1-13 which is an antibody or fragment thereof, a peptide, or a combination thereof.
15. The antagonist of any of paras. 1-14, wherein binding of the antagonist to c-met inhibits HGF dependent or independent c-met activation.
16. The antagonist of any of paras. 1-15, wherein binding of the antagonist to c-met in a cell inhibits proliferation, scattering, morphogenesis and/or motility of the cell.
17. The antagonist of any of paras. 1-16, wherein said antagonist comprises a peptide comprising at least a portion of c-met Sema domain or variant thereof.
18. The antagonist of para. 17, wherein said peptide comprises at least one of the sequences selected from the group consisting of LDAQT (SEQ ID NO: 1), LTEKRKKRS (SEQ ID NO:2), KPDSAEPM (SEQ ID NO:3) and NVRCLQHF (SEQ ID NO:4).
19. The antagonist of para. 17, wherein said peptide consists essentially of at least one of the sequences selected from the group consisting of LDAQT (SEQ ID NO: 1), LTEKRKKRS (SEQ ID NO:2), KPDSAEPM (SEQ ID NO:3) and NVRCLQHF (SEQ ID NO:4).
20. The antagonist of para. 17, wherein said peptide comprises the sequence LTEKRKKRS (SEQ ID NO: 2).
21. The antagonist of para. 17, wherein said peptide consists essentially of LTEKRKKRS (SEQ ID NO: 2).
22. A composition comprising the antagonist of any of paras. 1-21 and a carrier.
23. The composition of para. 22, wherein the carrier is pharmaceutically acceptable.
24. A nucleic acid encoding the antagonist of any of paras. 1-21, wherein the antagonist comprises a polypeptide.
25. The nucleic acid of para. 24, wherein the antagonist comprises an antibody or fragment thereof.
26. A vector comprising the nucleic acid of para. 24 or 25.
27. A host cell comprising the vector of para. 26.
28. An article of manufacture comprising:
   a container; and
   a composition contained within the container, wherein the composition comprises the antagonist of any of paras. 1-21.
29. The article of manufacture of para. 28 further comprising instructions for administering the antagonist to a subject.
30. A kit comprising:
   a first container comprising a composition comprising the antagonist of any of paras. 1-21; and
   a second container comprising a buffer.
31. The kit of para. 30, wherein the buffer is pharmaceutically acceptable.
32. The kit of para. 30, further comprising instructions for administering the antagonist to a subject.
33. A method of screening for or identifying a c-met antagonist, said method comprising:
   contacting a candidate substance with a target molecule comprising at least a portion of c-met Sema domain,
   whereby a substance that specifically binds said target molecule is selected as a c-met antagonist.
34. The method of para. 33, wherein the selected substance is contacted with a cell expressing c-met, and inhibition of c-met dimerization in the cell is detected or quantitated.
35. The method of para. 34, wherein inhibition of c-met dimerization is indicated by a decrease in amount of c-met activation.
36. The method of para. 35, wherein amount of c-met activation is indicated by amount of c-met associated cell signaling.
37. The method of para. 36, wherein said cell signaling is indicated by protein
   phosphorylation.
38. A method of modulating c-met activation in a subject, said method comprising administering to the subject a c-met Sema domain modulator, whereby c-met activity is modulated.
39. A method of inhibiting c-met activated cell proliferation, said method comprising contacting a cell or tissue with the antagonist of any of paras. 1-21, whereby cell proliferation associated with c-met activation is inhibited.
40. A method of treating a pathological condition associated with dysregulation of c-met activation in a subject, said method comprising to administering to the subject the antagonist of any of paras. 1-21, whereby c-met activation is inhibited.
41. A method of inhibiting the growth of a cell that expresses c-met or hepatocyte growth factor, or both, said method comprising contacting said cell with the antagonist of any of paras. 1-21 thereby causing an inhibition of growth of said cell.
42. A method of therapeutically treating a mammal having a cancerous tumor comprising cells that express c-met or hepatocyte growth factor, or both, said method comprising administering to said mammal a therapeutically effective amount of the antagonist of any of paras. 1-21, thereby effectively treating said mammal.
43. A method for treating or preventing a cell proliferative disorder associated with increased expression or activity of c-met or hepatocyte growth, or both, said method comprising administering to a subject in need of such treatment an effective amount of the antagonist of any of paras. 1-21, thereby effectively treating or preventing said cell proliferative disorder.
44. The method of para. 43, wherein said proliferative disorder is cancer.
45. A method for inhibiting the growth of a cell, wherein growth of said cell is at least in part dependent upon a growth potentiating effect of c-met or hepatocyte growth factor, or both, said method comprising contacting said cell with the antagonist of any of paras. 1-21, thereby inhibiting the growth of said cell.
46. A method of therapeutically treating a tumor in a mammal, wherein the growth of said tumor is at least in part dependent upon a growth potentiating effect of c-met or hepatocyte growth factor, or both, said method comprising contacting said cell with the antagonist of any of paras. 1-21, thereby effectively treating said tumor.
47. The method of para. 46, wherein said cell is a cancer cell.
48. The method of para. 47, wherein said cancer cell is further exposed to radiation treatment or a chemotherapeutic agent.
49. The method of para. 47, wherein said cancer cell is selected from the group consisting of a breast cancer cell, a colorectal cancer cell, a lung cancer cell, a papillary carcinoma cell, a prostate cancer cell, a lymphoma cell, a colon cancer cell, a pancreatic cancer cell, an ovarian cancer cell, a cervical cancer cell, a central nervous system cancer cell, an osteogenic sarcoma cell, a renal carcinoma cell, a hepatocellular carcinoma cell, a bladder cancer cell, a gastric carcinoma cell, a bead and neck squamous carcinoma cell, a melanoma cell and a leukemia cell.
50. The method of para. 47, wherein c-met or bepatoctye growth factor, or both, is more abundantly expressed by said cancer cell as compared to a normal cell of the same tissue origin.
51. The method of para. 46, wherein said hepatocyte growth factor is expressed in a different cell.
52. The method of para. 47, wherein activation of c-met is enhanced in said cancer cell as compared to a normal cell of the same tissue origin.
53. The method of para. 47 which causes the death of said cell.
54. The method of any of paras. 40-53 wherein in the absence of said antagonist c-met activation occurs independent of ligand.
55. The method of any of paras. 40-53 wherein in the absence of said antagonist c-met activation is ligand dependent.

## Claims

1. A c-met antagonist which is an antibody or fragment thereof, a peptide, or a combination thereof, that (i) disrupts c-met dimerization, or (ii) specifically binds a sequence in the c-met Sema domain.

2. The antagonist of claim 1 (i) which disrupts dimerization function of c-met Sema domain.

3. The antagonist of claim 1(i) which binds to c-met such that:
(i) c-met dimerization is disrupted; or
(ii) ability of c-met Sema domain to effect c-met dimerization is disrupted.

4. The antagonist of claim 1 which upon binding to a c-met molecule inhibits dimerization of said molecule, and, optionally, wherein said dimerization comprises homodimerization.

5. The antagonist of any one of claims 1-4 which:
(i) does not bind an HGF binding site on c-met;
(ii) does not substantially compete with hepatocyte growth factor (HGF) for binding to c-met;
(iii) does not substantially inhibit binding of hepatocyte growth factor to c-met;
(iv) competes with HGF for binding to c-met;
(v) binds to an epitope on c-met distinct from an epitope to which the monoclonal antibody produced by the hybridoma cell line deposited under American Type Culture Collection Accession Number ATCC HB11894 (hybridoma 1A3.3.13) or HB-11895 (hybridoma 5D5.11.6) binds; or
(vi) is not the monoclonal antibody produced by the hybridoma cell line deposited under American Type Culture Collection Accession Number ATCC HB-11894 (hybridoma 1A3.3.13) or HB-11895 (hybridoma 5D5.11.6).

6. The antagonist of any one of claims 1-5, wherein (i) binding of the antagonist to c-met inhibits HGF dependent or independent c-met activation, or (ii) binding of the antagonist to c-met in a cell inhibits proliferation, scattering, morphogenesis and/or motility of the cell.

7. The antagonist of any one of claims 1-6, wherein said antagonist comprises a peptide comprising at least a portion of c-met Sema domain or variant thereof.

8. The antagonist of claim 7, wherein said peptide:
(i) comprises at least one of the sequences selected from the group consisting of LDAQT (SEQ ID NO: 1), LTEKRKKRS (SEQ ID NO:2), KPDSAEPM (SEQ ID NO:3) and NVRCLQHF (SEQ ID NO:4);
(ii) consists essentially of at least one of the sequences selected from the group consisting of LDAQT (SEQ ID NO:1), LTEKRKKRS (SEQ ID NO:2), KPDSAEPM (SEQ ID NO:3) and NVRCLQHF (SEQ ID NO:4);
(iii) comprises the sequence LTEKRKKRS (SEQ ID NO:2); or
(iv) consists essentially of LTEKRKKRS (SEQ ID NO:2).

9. A composition comprising the antagonist of any one of claims 1-8 and a carrier, and, optionally, wherein the carrier is pharmaceutically acceptable.

10. A nucleic acid encoding the antagonist of any one of claims 1-8.

11. A vector comprising the nucleic acid of claim 10.

12. A host cell comprising the vector of claim 11.

13. An article of manufacture comprising:
a container; and
a composition contained within the container, wherein the composition comprises the antagonist of any of claims 1-8, and, optionally, further comprising instructions for administering the antagonist to a subject.

14. A kit comprising:
a first container comprising a composition comprising the antagonist of any of claims 1-8; and
a second container comprising a buffer, and, optionally, wherein the buffer is pharmaceutically acceptable, and, optionally, further comprising instructions for administering the antagonist to a subject.

15. A method of screening for or identifying a c-met antagonist, said method comprising:
contacting a candidate substance with a target molecule comprising at least a portion of c-met Sema domain,
whereby a substance that specifically binds said target molecule is selected as a c-met antagonist, and, optionally, wherein the selected substance is contacted with a cell expressing c-met, and inhibition of c-met dimerization in the cell is detected or quantitated.

16. The method of claim 15, wherein inhibition of c-met dimerization is indicated by a decrease in amount of c-met activation, and, optionally, wherein amount of c-met activation is indicated by amount of c-met associated cell signalling, and, optionally, wherein said cell signaling is indicated by protein phosphorylation.

17. The antagonist of any one of claims 1-8 for use in a method of:
(i) inhibiting c-met activated cell proliferation, said method comprising contacting a cell or tissue with the antagonist of any one of claims 1-8, whereby cell proliferation associated with c-met activation is inhibited;
(ii) inhibiting the growth of a cell wherein (a) the cell expresses c-met or hepatocyte growth factor, or both, or (b) growth of said cell is at least in part dependent upon a growth potentiating effect of c-met or hepatocyte growth factor, or both, said method comprising contacting said cell with the antagonist of any one of claims 1-8, thereby inhibiting the growth of said cell.

18. The antagonist of any one of claims 1-8 for use in a method of treating a pathological condition associated with dysregulation of c-met activation in a subject, said method comprising to administering to the subject the antagonist of any one of claims 1-8, whereby c-met activation is inhibited.

19. The antagonist for use according to claim 18, wherein the pathological condition is a cell proliferative disorder associated with increased expression or activity of c-met or hepatocyte growth, or both, said method comprising administering to a subject in need of such treatment an effective amount of the antagonist of any of claims 1-8, thereby effectively treating or preventing said cell proliferative disorder.

20. The antagonist for use according to claim 19, wherein said proliferative disorder is cancer.

21. The antagonist for use according to claim 18, wherein the pathological condition is a tumor in a mammal wherein the tumor comprises cells (a) that express c-met or hepatocyte growth factor, or both, or (b) growth of which is at least in part dependent upon a growth potentiating effect of c-met or hepatocyte growth factor, or both, said method comprising administering to said mammal a therapeutically effective amount of the antagonist of any one of claims 1-8, thereby effectively treating said mammal.

22. The antagonist for use according to claim 21, wherein said cell is a cancer cell.

23. The antagonist for use according to claim 22, wherein said cancer cell (i) is further exposed to radiation treatment or a chemotherapeutic agent, or (ii) is selected from the group consisting of a breast cancer cell, a colorectal cancer cell, a lung cancer cell, a papillary carcinoma cell, a prostate cancer cell, a lymphoma cell, a colon cancer cell, a pancreatic cancer cell, an ovarian cancer cell, a cervical cancer cell, a central nervous system cancer cell, an osteogenic sarcoma cell, a renal carcinoma cell, a hepatocellular carcinoma cell, a bladder cancer cell, a gastric carcinoma cell, a bead and neck squamous carcinoma cell, a melanoma cell and a leukemia cell.

24. The antagonist for use according to claim 22, wherein (i) c-met or hepatocyte growth factor, or both, is more abundantly expressed by said cancer cell as compared to a normal cell of the same tissue origin, (ii) said hepatocyte growth factor is expressed in a different cell, (iii) activation of c-met is enhanced in said cancer cell as compared to a normal cell of the same tissue origin, or (iv) the antagonist causes the death of said cell.

25. The antagonist for use according to any one of claims 17-24 wherein in the absence of said antagonist c-met activation (i) occurs independent of ligand or (ii) is ligand dependent.
